# EUROPEAN PATENT APPLICATION

(11) **EP 4 056 052 A1**
(43) Date of publication of application: **14.09.2022**
(21) Application number: 21162450.7
(22) Date of filing: 12.03.2021
(51) Int. Cl.: A23L 33/135, A23L 33/125

(54) **GOS PRE-CONDITIONING L. REUTERI AND GOS IN FINAL FORMULATION**

(71) Applicant: Biogaia AB, 112 27 Stockholm (SE)
(72) Inventor: ROOS, Stefan, 757 54 Uppsala (SE); HORCAJADA, Marie Noëlle, 01170 ECHENEVEX (FR); BONNET, Nicolas, 74380 NANGY (FR); SABATIER, Magalie, 1000 LAUSANNE 26 (CH); BOURQUI, Bertrand, 3013 BERN (CH); PRIOULT, Guénolée Eliane Marie, 3007 BERN (CH); DE BRUYN, Florac, 1006 LAUSANNE (CH)
(74) Representative: Barker Brettell Sweden AB

(57) **Abstract**

The invention herein relates generally to enhancing the survival and activity of probiotic *Lactobacillus reuteri* strains in mammals by pre-conditioning *L. reuteri* with GOS during cultivation and to a surprisingly high synbiotic effect of such a pre-conditioned probiotic *L. reuteri* strain when administered together with GOS further added in the composition. The invention therefore comprises methods for cultivating and manufacturing probiotic *L. reuteri* strains, and preparing products containing such pre-conditioned strains in combination with additional amounts of GOS. In more detail the present invention relates to a composition comprising a probiotic *L. reuteri* strain, obtained by a step of growing the bacteria in a medium comprising GOS ("pre-conditioning"), and administering the pre-conditioned bacteria together with further GOS in the final product. The inventors have found that such method induces surprisingly high and unexpected synbiotic beneficial effects of the probiotic bacteria in the gastrointestinal tract. The composition also induces an increased calcium and iron solubility.

## Description

### TECHNICAL FIELD

The invention herein relates generally to enhancing the survival and activity of probiotic *Lactobacillus reuteri* strains in mammals by pre-conditioning *L. reuteri* with galacto-oligosaccharides (GOS) during cultivation and to a surprisingly high synbiotic effect of such a pre-conditioned probiotic *L. reuteri* strain when administered together with GOS further added in the composition. The invention therefore comprises methods for cultivating and manufacturing probiotic *Lactobacillus reuteri* strains, and preparing products containing such pre-conditioned strains in combination with additional amounts of GOS. In more detail the present invention relates to a composition comprising a probiotic *L. reuteri* strain, obtained by a step of growing the bacteria in a medium comprising GOS ("pre-conditioning"), and administering the pre-conditioned bacteria together with further GOS in the final product. The inventors have found that such a method induces surprisingly high and unexpected synbiotic beneficial effects in the gastrointestinal tract. The composition also induces an increased iron and calcium solubility.

### BACKGROUND

Probiotics are defined as "live microorganisms that, when administered in adequate amounts, confer a health benefit on the host." This is the widely accepted scientific definition around the world (see e.g. Hill et al. 2014; Nat. Rev. Gastro. & Hepathology, Vol 11). Probiotic products (which is usually dietary supplements or foods) may be recommended for different conditions or symptoms an individual is experiencing. Lactic acid producing bacteria, such as Lactobacilli, are commonly used as probiotics in various types of foods, for example yoghurt. Growth and colonization of harmful microorganisms can be prevented by such lactic acid producing bacteria through their own colonization inside the intestinal system, through competition of available nutrients and/or through production of specific substances, such as hydrogen peroxide, bacteriocins and/or organic acids, including lactic and acetic acid, that lowers the intestinal pH. It is well established that interactions between host and gut microbes are fundamental to health and well-being of the host. Intestinal microbiota generates metabolites that provide the host with nutrients but may also be involved in the immune response and in regulation and development of the host's immune system as well as reducing inflammation and preventing allergic responses.

Prebiotics are compounds that induce the growth or activity of beneficial microorganisms, such as bacteria and fungi, by selectively stimulating their growth and/or activity. In the gastrointestinal tract, prebiotics can therefore alter the composition of the gut microbiome. Dietary prebiotics are typically nondigestible (see e.g., https://en.wikipedia.org/wiki/Dietary_fiber) fiber compounds that pass undigested through the upper part of the gastrointestinal tract and stimulate the growth or activity of advantageous bacteria that colonize the large bowel by acting as their substrate. Various compounds have been tested to determine their function as prebiotics. Fructo-oligosaccharides (FOS), galacto-oligosaccharides (GOS), and trans-galacto-oligosaccharides (TOS) are the most common prebiotics. Consuming certain prebiotics can, thus, improve immunity functions by increasing the population of microorganisms associated with human health, and animal and human studies have shown that prebiotics can decrease the population of harmful bacteria. Prebiotics are, thus, ingredients, providing health benefits when fermented by the native microflora in the intestine of a subject or by probiotic bacteria ingested simultaneously with the prebiotics.

Combining particular types of prebiotics and probiotics is generally known to give rise to certain synergistic effects. In particular, the effect of combined prebiotic GOS and probiotic microorganisms, such as Lactobacillus and Bifidobacteria, has been documented (see e.g. YUTAKA KANAMORI, MD et al., Digestive Diseases and Sciences, Vol. 46, No. 9 (September 2001), pp. 2010-2016 (^{©} 2001)). It has been demonstrated that the synbiotic effect between prebiotics and probiotics is due to the consumption of the prebiotic by the probiotic within the gastrointestinal (GI) tract of the subject.

The manufacturing procedure of probiotic lactic acid producing bacteria is typically standardized and involves a step of fermenting the bacteria in a growth medium comprising a carbohydrate source, such as a sugar, for example glucose, fructose, sucrose, lactose or dextrose. Following the fermentation, the probiotic bacteria are usually protected and frozen or freeze-dried and packaged into a finished product.

The ingestion of probiotic lactic acid bacteria has various effects *in vivo.* One such important effect is the bio-accessibility of essential minerals, which can be significantly influenced by the presence of such probiotic lactic acid bacteria. Although the content of minerals in the body depends primarily on their supply from food intake and they are absorbed mainly in the GI tract, mere provision of minerals via food intake does not necessarily lead to a sufficient mineral bio-accessibility. The main factors affecting mineral bio-accessibility are the content of minerals in the food, synergistic and antagonistic interactions between minerals in the food and in the GI tract, the presence of complexing or chelating compounds in the food, and the health state of the organism and its age. Also, the intestinal microflora, probiotics and prebiotics significantly influence the bio-accessibility of minerals and can thereby increase or decrease the absorption of such minerals.

Minerals are essential for all living species, even though the specific requirements differ between species. Minerals have a great number of important functions in the human organism and include e.g., iron and calcium, among many others. Mineral deficiencies, i.e., low or suboptimal amounts of such minerals in the body, typically by ingesting minerals below recommended RDA amounts, can lead to diseases and so can mineral excess. It is therefore pivotal to obtain the correct amounts of minerals at the correct ratios for optimal health. Most natural diets will provide these minerals in appropriate balances, but there are situations when this is not enough to maintain health.

Calcium for example, is the most abundant mineral in the human body and is an important bone component and an enzyme activator. Calcium also takes part in conduction of bioelectric impulses, blood coagulation, muscle contraction, inflammation and hormonal secretion. Calcium is absorbed in the small intestine in the presence of the active form of vitamin D (calcitriol). Hypocalcemia, commonly known as calcium deficiency, can lead to various side effects, conditions or even severe diseases. Calcium deficiency is most common in older adults, teenagers, and people who are overweight. The reported prevalence for permanent hypocalcemia ranges from 0.4% and 33% with renal failure remaining the most common type of hypocalcemia, followed by vitamin D deficiency. While the incidence of hypocalcemia is difficult to quantify, global estimates in accounted for 3.4 billion people at risk for calcium deficiency. Usually, average calcium levels are kept through the actions of the parathyroid hormone (PTH), the kidneys, and the intestines. Calcium deficiency can lead to various conditions or disorders, such as muscle problems, e.g., cramps, muscle spasms, and aches, to fatigue, including insomnia, sleepiness, and extreme fatigue, to skin and nail symptoms, such as eczema, to psoriasis, redness, itchiness, and skin blisters, osteoporosis and osteopenia, to painful premenstrual syndrome, dental problems, depression, etc. Populations at risk for calcium deficiency, include particularly pregnant women (especially in the last trimester), lactating women, postmenopausal women, and, possibly, elderly men. However, calcium deficiency (hypocalcemia) is also serious problem in early infancy. Hypocalcemia in early infancy is usually termed neonatal hypocalcemia. Neonatal hypocalcemia particularly occurs in the first 2 to 3 days of a baby's life. To some degree neonatal hypocalcemia is part of a normal developmental process and can be efficiently treated by normal nutritional intervention. A late hypocalcemia starts in the first week or weeks after birth and is less likely to go away without nutrition support and can be efficiently treated by corresponding supplements or fortified infant formulae.

Iron for instance is absorbed in the duodenum and the small intestine in the form of Fe²⁺. Iron is a component of hemoglobin, which is responsible for the transport of oxygen, and myoglobin, and it is also a part of many enzymes such as catalase, peroxidase and cytochromes. In addition, iron is very important in maintaining several other body functions, such as in the maintenance of healthy cells, skin, hair, and nails. Cells that line the gastrointestinal membranes absorb iron from the ingested food. Iron can be stored in the liver and released as needed to make new red blood cells in the bone marrow. Iron deficiency is a condition, which occurs when the body doesn't have enough of the mineral iron and often this leads to abnormally low levels of red blood cells and a condition referred to as iron deficiency anemia. As a result, iron deficiency anemia may leave you tired and short of breath. Iron deficiency can be caused by blood loss, lack of dietary iron, an inability to absorb iron, and during pregnancy. Several risk factors and risk groups are associated with iron deficiency anemia such as women with heavy menstruation or pregnancy. Another group at risk for iron deficiency is infants, especially those with a low birth weight or which are born prematurely, or those infants who don't get enough iron from breast milk or formula. In general, children need extra iron during growth spurts. Furthermore, a poor diet or certain intestinal diseases that affect how the body absorbs iron can also cause and be a risk factor for iron deficiency anemia. Iron deficiency is thus very common in certain groups of people and even if it, in most cases, does not lead to any severe complications; untreated iron deficiency can have detrimental effects. Taken together, there is a need and room for new methods and safe products that can increase the iron levels in patients with iron deficiency and that can be used in treatment of an iron deficiency and diseases or disorders related to iron deficiency.

Iron and iron levels are of particular importance in pregnant subjects, fetus and babies/infants. Infants and toddlers are vulnerable to iron deficiencies, especially in developing countries, due to the high dietary requirement in the weaning period in proportion to body size. During this period, iron is a critical nutrient for development, particularly for brain and muscle tissues. Prevalence of iron deficiency is the highest at 1 to 3 years of age. The prevalence in Europe ranges between 5 and 20% (Domellöf, M et al, D. (2014)), and can be higher for Asian countries, up to 35% (Duggan, MB et al, S. (1991)).

Recent studies have produced new information on microbiota - mineral interactions and have mainly concerned the impact of intestinal bacteria on the metabolism of calcium and iron. Given the aging population and the rising prevalence of osteoporosis among postmenopausal women and men, the new data on calcium metabolism and hence on bone metabolism and its improvement are crucial to the development of the field. In contrast, the results of research into adverse interactions between iron and the intestinal microbiota show that there is a crucial need for further studies on the bio-accessibility of this mineral in order to prepare new recommendations regarding iron supplementation.

Also, a common problem with oral administration of probiotic bacteria is that they arrive at their destined location in the gastrointestinal tract in insufficient amounts and/or that the activity of the probiotic bacteria in these locations of the intestinal tract, where they assert their effects, is inadequate. The survival, viability and engraftment of the probiotic bacteria in the gastrointestinal tract is thus a significant challenge for those who manufacture probiotic products. As a consequence, the dosage of probiotic bacteria has to be increased and/or more frequent administration is required to obtain the desired probiotic effect. This further leads to problems with unnecessary costs, undesirable frequency of intake and possibly also decreased health benefits when the probiotic bacteria do not sufficiently provide the desired effects. Thus, it is desired to improve the probiotic effect without increasing the dosage.

### SUMMARY OF THE INVENTION

It is an object of the current invention to provide more efficient means, methods and therapies of improving the survival and probiotic effects of probiotic microorganisms, such as lactobacilli, when administering such probiotics to a subject or a patient in need thereof. Moreover, it is a particular object to prevent and/or treat mineral deficiency in a subject or in a patient in need thereof, particularly mineral deficiencies of minerals, e.g. selected from iron and/or calcium.

These objects are solved by the current invention as addressed below.

The present invention is defined below and in the independent claims. Further embodiments of the invention are defined in the specification and in the dependent claims.

In a first aspect, the invention provides a method for preparing a probiotic composition, characterized in that the method comprises the steps:
a) pre-conditioning of a probiotic *Lactobacillus reuteri* (*L. reuteri)* strain following the steps:
   i) cultivating a probiotic *L. reuteri* strain in the presence of galacto-oligosaccharide (GOS) in a growth medium, thereby pre-conditioning the probiotic *L. reuteri* strain; and
   ii) optionally harvesting the pre-conditioned probiotic *L. reuteri* strain from the growth medium;
b) preparing a composition comprising the pre-conditioned probiotic *L. reuteri* strain obtained from step a)i) or aii) and adding GOS to the composition.

The composition prepared by the inventive method comprises the pre-conditioned probiotic *L. reuteri* strain and GOS.

In step a)i) of the inventive method, pre-conditioning of a probiotic *L. reuteri* strain occurs by cultivating or culturing a probiotic *L. reuteri* strain in the presence of GOS in a growth medium, thereby pre-conditioning the probiotic *L. reuteri* strain. Cultivation of a probiotic *L. reuteri* strain in the presence of GOS leads to formation of the pre-conditioned probiotic *L. reuteri* strain.

In the inventive method, the GOS may be added in step a)i) to the growth medium in an amount of at least 0.2 wt%, preferably at least 0.5 wt%, even more preferably at least 0.75 wt% of GOS in the growth medium, and preferably at most 8 wt% of GOS, such as at most 7 wt% GOS, at most 6 wt% GOS, or even at most 5 wt% GOS, more preferably at most 4 wt% of GOS or even at most 3% GOS, at most 2 wt% of GOS, or at most 1 wt% of GOS in the growth medium, preferably calculated on basis of the growth medium (% w/w). Any combination of these upper and lower ranges is encompassed herewith. More preferably, GOS is added to the growth medium in a range of 0.2 wt% to 8 wt% or even 0.2 wt% to 7 wt%, or 0.2 wt% to 6 wt%, more preferably in a range of 0.5 wt% to 8 wt% or even 0.5 wt% to 7 wt%, or 0.5 wt% to 6 wt%, even more preferably in a range of 0.75 wt% to 8 wt% or even 0.75 wt% to 7 wt%, or 0.75 wt% to 6 wt%, and most preferably in a range of 0.75 wt% to 7 wt%, in a range of 0.75 wt% to 6 wt% or even in a range of 0.75 wt% to 5 wt%, such as a range of 0.75 wt% to 3 wt% or 0.75 wt% to 4 wt%, preferably calculated on basis of the growth medium (% w/w). Addition of GOS may occur preferably at the start, in the middle or at the end of cultivation of a probiotic *L. reuteri* strain in step a)i), and/or at once, stepwise or continuously.

Moreover, GOS may be preferably added in step b) to the pre-conditioned probiotic *L. reuteri* strain obtained from step a) in an amount of at least 1 or 2 g per daily dose, preferably at least 3 g per daily dose, likewise preferably between 2 to 12 g per daily dose, or between 2 to 10 g per daily dose, between 2 to 8 g per daily dose, or between 2 to 7 g per daily dose, more preferably between 3 to 12 g per daily dose, likewise more preferably between 3 and 10 g per daily dose, such as between 3 to 8 g per daily dose, between 3 to 7 g per daily dose, between 3 to 6 g per daily dose, or about 2, 3, 4, 5, 6, 7, 8, 9, 10, 11, or 12 g per daily dose or any range formed thereby.

Moreover, the pre-conditioned probiotic *L. reuteri* strain obtained in step b) from step a)i) or a)ii) is preferably present in the composition in an amount of between 10³ cfu to 10¹² cfu per daily dose, likewise preferably in an amount of between 10⁴ cfu to 10¹¹ cfu per daily dose, more preferably in an amount of between 10⁵ cfu to 10⁹ cfu per daily dose, even more preferably in an amount of between 10⁶ cfu to 10⁹ cfu per daily dose or in an amount of 10⁸ cfu to 10¹⁰ cfu per daily dose, most preferably in an amount of around 10⁸ total cfu per daily dose, including an amount of 10⁷ to 10⁹ or 10⁸ to 10⁹ cfu per daily dose

Moreover, the GOS added to the growth medium in step a)i) of the inventive method and/or to the composition comprising the pre-conditioned probiotic *L. reuteri* strain prepared in step b) of the inventive method preferably may have a degree of polymerization (DP) of 3-8. The cultivation in step a)i) of the inventive method may occur over a period of 5 to 18 hours, preferably 7 to 14 hours, and more preferably 10 to 13 hours, and even more preferably about 12 hours. According to a preferred embodiment, cultivation in step a)i) of the inventive method occurs at a temperature of between 25 to 45°C, preferably between 30 and 40°C, most preferably at a temperature of about 37°C. The pH during pre-conditioning of probiotic *L. reuteri* strain during cultivation step a) may be either not controlled or set to a constant pH value throughout the cultivation step a)i), typically to be monitored and adjusted during cultivation step a). Accordingly, the pH may be between 3.0 and 7.0, particularly when pH is not controlled during pre-conditioning of probiotic *L. reuteri* strain. Alternatively, if pH is controlled during cultivation step a)i), pH may be adjusted to a range of about 5.0 to 7.0, e.g., about 5.0 to 6.0, about 5.5 to 6.5 (e.g., about 6.0±0.2) or about 6.0 to 7.0 (e.g., about 6.6±0.2). For instance, pH may be adjusted to a range of about 5.5 to 6.5, preferably pH may be adjusted to a range of about 6.0±0.4, and more preferably pH may be adjusted to a range of about 6.0±0.2. Alternatively, pH is adjusted to about 6.0 to 7.0, preferably to about 6.6±0.4, and more preferably to about 6.6±0.2. It is added the inventive method for a prebiotic composition may comprise a further step a)iii) of washing the pre-conditioned probiotic *L. reuteri* strain after harvesting step a)ii), preferably to remove trace amounts of growth medium. The inventive method may also comprise a further step a)iv) of drying the harvested pre-conditioned probiotic *L. reuteri* strain, step a)iv) either carried out after washing step a)iii) or after harvesting step a)ii). The drying step a)iv) may be carried out using spray-drying, fluid bed drying, air convective drying, atmospheric drying, roller drying or freeze drying, lyophilizing, and more preferably spray-drying or freeze drying. In case of need, the drying step may be preceded by treating the harvested pre-conditioned probiotic *L. reuteri* strain with a protectant, a cryoprotectant, a lyoprotectant and/or a carrier. Alternatively, or additionally, the pre-conditioned *L. reuteri* strain obtained after any of steps a)i, a)ii) or a)iii) or a)iv) may be encapsulated for further processing, preferably prior to the drying step a)iv). Particularly preferably, the probiotic *L. reuteri* strain is at least one *L. reuteri* strain or multiple *L. reuteri* strains selected from the group comprising or consisting of *L. reuteri* strain as deposited under DSM 17938, *L. reuteri* strain as deposited under ATCC PTA 5289, *L. reuteri* strain as deposited under ATCC PTA 6475, *L. reuteri* as deposited under DSM 32846, *L. reuteri* as deposited under DSM 32848, *L. reuteri* as deposited under DSM 32849, *L. reuteri* as deposited under DSM 27131, *L. reuteri* as deposited under DSM 32465 and *L. reuteri* strain as deposited under ATCC PTA 4659, preferably the probiotic *L. reuteri* strain is *L. reuteri* strain as deposited under DSM 17938.

According to a second aspect, the invention provides a composition comprising an effective amount of a pre-conditioned probiotic *L. reuteri* strain, and additionally an effective amount of a GOS, wherein the composition has been prepared preferably according to a method as defined herein, preferably according to the first aspect of the invention. The inventive composition may comprise the pre-conditioned probiotic *L. reuteri* strain in an amount of between 10³ cfu to 10¹² cfu, typically in an amount of between 10⁴ cfu to 10¹¹ cfu per daily dose, preferably in an amount of between 10⁵ cfu to 10¹⁰ cfu per daily dose, or 10⁵ cfu to 10⁹ cfu per daily dose, likewise preferably in an amount of between 10⁶ cfu to 10⁹ cfu per daily dose, 10⁶ cfu to 10⁸ cfu per daily dose or in an amount of 10⁸ cfu to 10¹⁰ cfu per daily dose, more preferably around 10⁷ cfu to 10⁹ cfu per daily dose. A preferred daily dose is around 10⁸ total cfu's per daily dose, e.g., 10⁷ to 10⁹ cfu per daily dose or 10⁸ to 10⁹ cfu per daily dose. The composition furthermore may comprise GOS in an amount of at least 1 or 2 g per daily dose, preferably at least 3 g per daily dose, likewise preferably between 2 to 12 g per daily dose, or between 2 to 10 g per daily dose, between 2 to 8 g per daily dose, or between 2 to 7 g per daily dose, more preferably between 3 to 12 g per daily dose, likewise more preferably between 3 and 10 g per daily dose, such as between 3 to 8 g per daily dose, between 3 to 7 g per daily dose, between 3 to 6 g per daily dose, or about 2, 3, 4, 5, 6, 7, 8, 9, 10, 11, or 12 g per daily dose or any range formed thereby.

As outlined herein, the inventive composition also allows or is for improving the probiotic effect, e.g., of an *L. reuteri* strain in the intestinal tract of a subject, for increasing mineral bio-accessibility in the intestinal tract of a subject, for increasing iron and calcium solubility and/or absorption in the intestinal tract of a subject, for increasing metabolic activity of the gut microbiome, for increasing lactic acid and/or acetic acid in the intestinal tract of a subject, for promoting bacterial survival and persistence in the intestinal tract of a subject, and/or for promoting a healthy microbiota in the intestinal tract of a subject. In one embodiment the inventive composition improves the survival and persistence of the *L*. *reuteri* in the gastrointestinal tract of a subject.

The inventive composition may be furthermore a solid or liquid, and/or may be present in form of a powder, a tablet, a capsule, or may be in form of an oil formulation, an emulsion, an oil-in-water emulsion (o/w emulsion), or a water-in-oil emulsion (w/o emulsion). Additionally, without being limiting thereto, the inventive composition may be in an administrable form, preferably selected from the group consisting of nutritional compositions, pharmaceutical formulations, dietary supplements, functional food products, functional beverage products, and combinations thereof, e.g., a dairy product, a milk-based product, a whey protein-based beverage.

In a third aspect, the invention provides a non-therapeutic use of the inventive composition comprising an effective amount of a pre-conditioned probiotic *L. reuteri* strain supplemented with an effective amount of GOS, the composition being prepared according to the invention or as described herein, for increasing or boosting the probiotic effect, e.g. of the probiotic *L. reuteri* strain in the digestive tract of a healthy subject. The increase of the probiotic effect may be manifested e.g., in improving the *L. reuteri* metabolic activity of the gut microbiome in the intestinal tract of a healthy subject, increasing lactic acid and/or acetic acid production in the intestinal tract of a subject, promoting bacterial survival and persistence of the probiotic *L. reuteri* strain, promoting a healthy microbiota in a healthy subject, increasing bio-accessibility of minerals in the intestinal tract of the healthy subject, and/or increasing iron and calcium solubility and/or absorption in the intestinal tract of a subject. In one embodiment, the increase of the probiotic effect may be manifested e.g., in improving the *L. reuteri* metabolic activity of the gut microbiome in the intestinal tract of a healthy subject, increasing lactic acid and/or acetic acid production in the intestinal tract of a subject, promoting a healthy microbiota in a healthy subject, increasing bio-accessibility of minerals in the intestinal tract of the healthy subject, and/or increasing iron and calcium solubility and/or absorption in the intestinal tract of a subject.

The inventive composition for non-therapeutic use preferably increases mineral solubility, accessibility and/or absorption of minerals in the intestinal tract of the healthy subject, increases survival and/or engraftment and/or viability of an *L. reuteri* strain in the intestinal tract of the healthy subject, and/or increases the lactic acid/acetic acid production in the microbiota or intestinal tract of the healthy subject. The inventive composition for non-therapeutic use preferably leads to an improvement of bone health, bone formation, teeth conditions, teeth growth, muscular functions, cognitive functions, immune functions and/or general growth and development (especially for children/infants), preferably due to an increase of mineral solubility and/or mineral absorption, in the intestinal tract of the healthy subject. Particularly relevant minerals in this context include calcium and/or iron, preferably calcium and iron. Moreover, the healthy subject is typically selected from an athlete, a child, a toddler or an infant, an adult, an elderly, a pregnant woman, a vegetarian, a lactating woman, a companion animal, a cat, or a dog, or a bird, such as poultry. A healthy subject in this context does usually not suffer from any disease, particularly any diseases related to mineral deficiency. The inventive composition may be solid or liquid, and/or is present in form of a powder, a tablet, a capsule, or may be in form of an oil formulation, an emulsion, an oil-in-water emulsion (o/w emulsion), or a water-in oil emulsion (w/o emulsion). Moreover, the inventive composition may be in an administrable form, preferably selected from the group consisting of nutritional compositions, pharmaceutical formulations, dietary supplements, functional food products, functional beverage products, and combinations thereof, e.g., a dairy product, a milk-based product, a whey protein-based beverage. As before, the *L. reuteri* strain is particularly preferable at least one *L. reuteri* strain or multiple *L. reuteri* strains selected from the group comprising or consisting *of L. reuteri* strain as deposited under DSM 17938, *L. reuteri* strain as deposited under ATCC PTA 5289, *L. reuteri* strain as deposited under ATCC PTA 6475, *L. reuteri* as deposited under DSM 32846, *L. reuteri* as deposited under DSM 32848, *L. reuteri* as deposited under DSM 32849, *L. reuteri* as deposited under DSM 27131, *L. reuteri* as deposited under DSM 32465 and *L. reuteri* strain as deposited under ATCC PTA 4659, preferably the probiotic *L. reuteri* strain is *L*. *reuteri* strain as deposited under DSM 17938.

According to a fourth aspect, the invention provides a composition comprising an effective amount of a pre-conditioned probiotic *L. reuteri* strain and an effective amount of GOS, preferably as prepared according to the invention or as described herein, for use as a medicament.

According to a fifth aspect, the invention provides a composition comprising an effective amount of a pre-conditioned probiotic *L. reuteri* strain and an effective amount of GOS, preferably as prepared according to the invention or as defined herein, for (therapeutic) use in the prevention and/or treatment of a disease in a patient in need thereof.

In the above mentioned fourth and fifth aspect, the patient is preferably at risk of developing mineral deficiency or is a subject suffering from mineral deficiency.

Moreover, the pre-conditioned probiotic *L. reuteri* strain and additionally GOS significantly increase the probiotic effect of a probiotic *L. reuteri* strain in a surprising and synbiotic manner. Such an increase of the probiotic effect is manifested e.g. as an increase of mineral bio-accessibility, preferably an increase of mineral solubility and/or mineral absorption in the intestinal tract of the patient, such as an increase iron and/or calcium in the intestinal tract of the patient; an increase of metabolic activity of the gut microbiome, exemplified by an increase of the lactic acid/acetic acid production in the intestine of the patient, and/or an increase of the probiotic *L. reuteri* strain survival and persistence in the intestinal tract of a subject. In these uses, the mineral is typically selected from magnesium, calcium and/or iron, preferably calcium and iron. The inventive compositions may be also for use in the prevention and/or treatment of iron deficiency, preferably iron deficiency anemia, in a patient in need thereof.

Alternatively, or additionally, the compositions may be also for use in the prevention and/or treatment of calcium deficiency in a patient in need thereof. Moreover, the inventive compositions may be for use in the prevention and/or the treatment of bone loss and/or bone loss related disease in a patient in need thereof. The inventive composition may be applied to a patient in need thereof, being a mammal, preferably selected form a child, a toddler or an infant, an elderly, a pregnant woman, a vegetarian, a lactating woman, a companion animal, a cat, or a dog, or a bird, such as poultry. A patient in need thereof is typically a subject either being at risk of developing mineral deficiency or having developed a mineral deficiency or having any further disease or condition. The inventive compositions applied for such uses may be solid or liquid, and/or may be present in form of a powder, a tablet, a capsule, or may be in form of an oil formulation, an emulsion, an oil-in-water emulsion (o/w emulsion), or a water-in oil emulsion (w/o emulsion). The inventive composition for such uses may also be in an administrable form, preferably selected from the group consisting of nutritional compositions, pharmaceutical formulations, dietary supplements, functional food products, functional beverage products, and combinations thereof, e.g., a dairy product, a milk-based product, a whey protein-based beverage. As before, the *L. reuteri* strain is particularly preferable at least one *L. reuteri* strain or multiple *L. reuteri* strains selected from the group comprising or consisting of *L. reuteri* strain as deposited under DSM 17938, *L. reuteri* strain as deposited under ATCC PTA 5289, *L. reuteri* strain as deposited under ATCC PTA 6475, *L. reuteri* as deposited under DSM 32846, *L. reuteri* as deposited under DSM 32848, *L*. *reuteri* as deposited under DSM 32849, *L. reuteri* as deposited under DSM 27131, *L. reuteri* as deposited under DSM 32465 and *L. reuteri* strain as deposited under ATCC PTA 4659, preferably the probiotic *L. reuteri* strain is *L. reuteri* strain as deposited under DSM 17938.

Further preferred embodiments are described herein below and in the dependent claims.

### BRIEF DESCRIPTION OF THE DRAWINGS

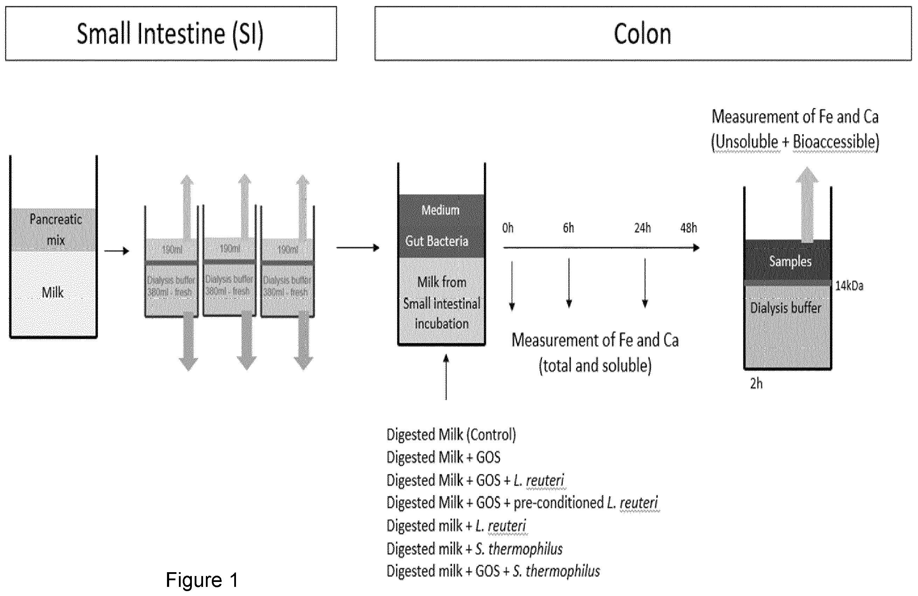

- **Figure 1:**: shows the short SHIME colonic in vitro model used to assess the impact of probiotic strains on microbiome metabolism and engraftment of probiotic strains.

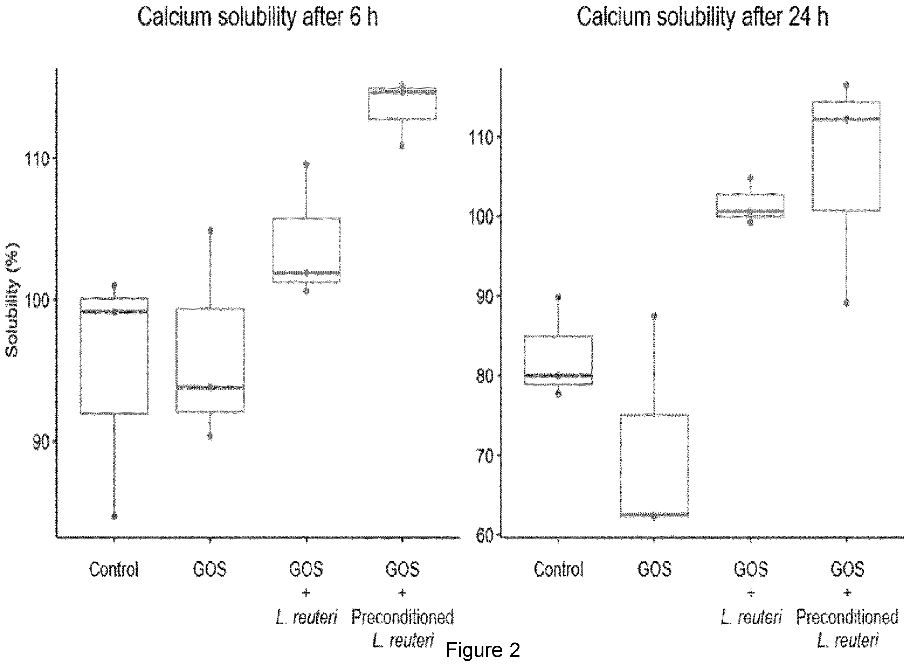

- **Figure 2:**: shows calcium solubility measured at 6 h and 24 h in the colon. As can be seen in Figure 2, pre-conditioned probiotic *L. reuteri* strain boosts calcium solubility in colon at both 6 h and 24 h in presence of GOS. This suggests that a pre-conditioned *L. reuteri* strain combined with GOS can be used to promote calcium absorption in a subject in need thereof.

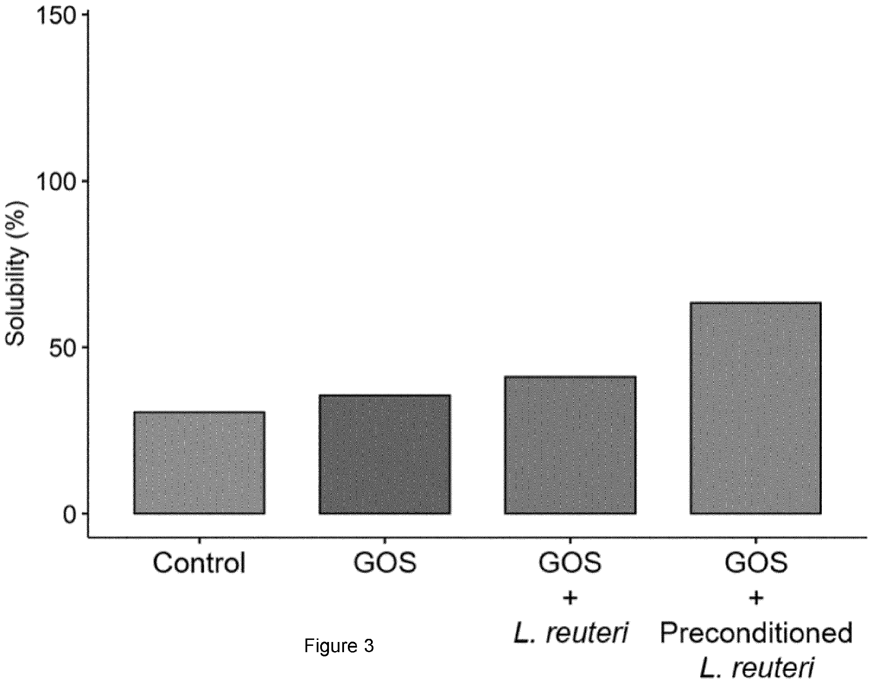

- **Figure** 3:: shows the improved iron solubility (bio-accessibility) in the colon at 48 h. GOS and pre-conditioned *L. reuteri* boost iron solubility (bio-accessibility) in the colon suggesting that the combination of both GOS and pre-conditioned *L. reuteri* can promote iron absorption in a subject in need thereof.

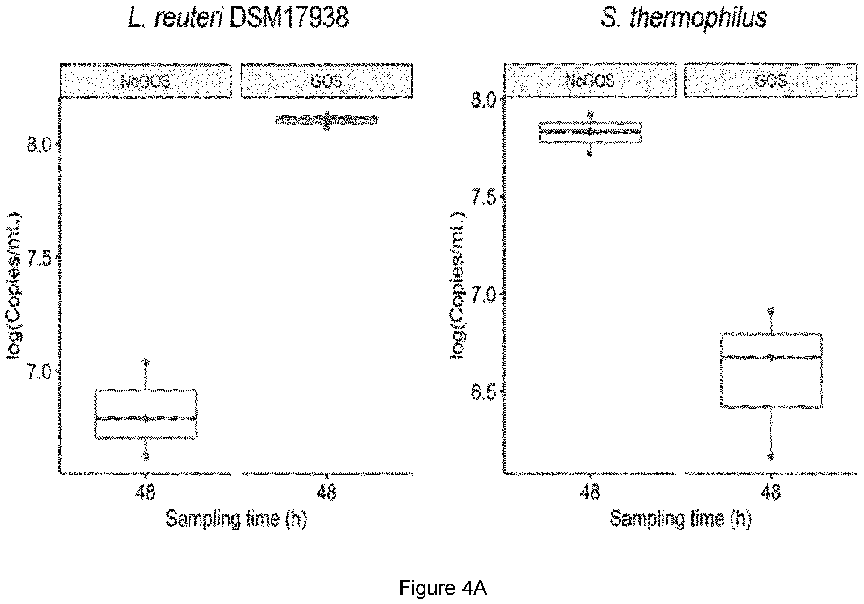

- **Figure 4A:**: shows that survival of *L. reuteri* DSM17938 and *Streptococcus thermophilus* NCC2496 in presence or absence of GOS in the colon after 48h incubation, particularly a positive effect of GOS on *L. reuteri* survival and negative effect of GOS on *Streptococcus thermophilus* survival.

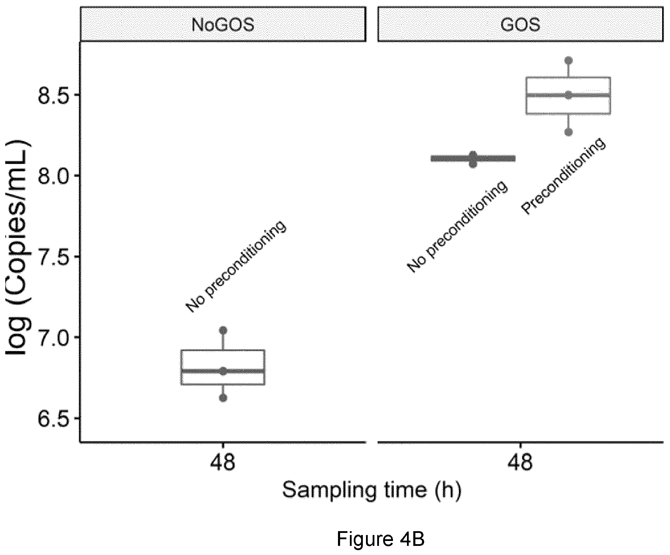

- **Figure 4B:**: shows that the survival of pre-conditioned *L. reuteri* DSM17938 is better than non-pre-conditioned *L. reuteri* strain in presence of GOS

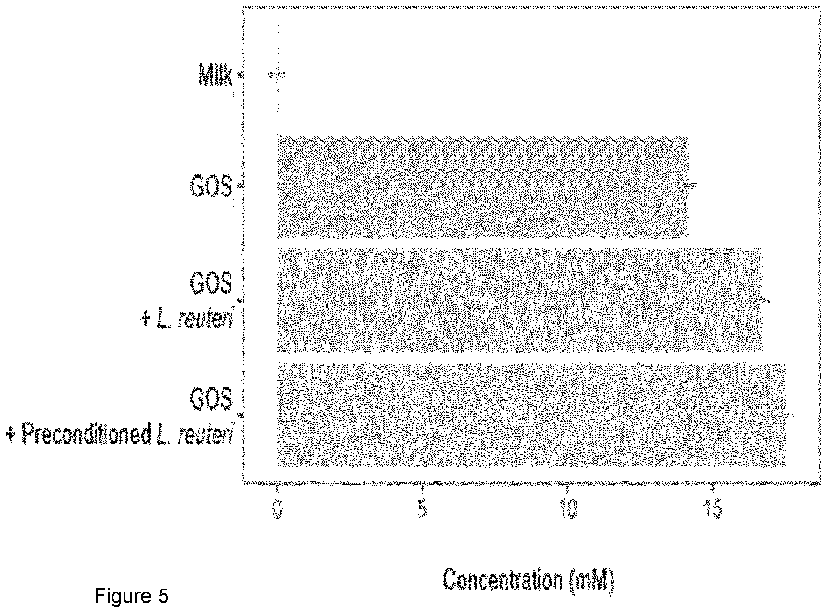

- **Figure 5:**: shows the lactic acid production (difference between the level at 48 h and 6 h). GOS alone induces a dramatic increase of lactic acid production in the colon. Addition of *L*. *reuteri* in addition to GOS further enhances the lactic acid production. Pre-conditioned *L. reuteri* in combination of GOS boosts even more than *L. reuteri* (not pre-conditioned) the production of lactic acid in the colon showing an overall increase of the metabolic activity of the microbiota. Lactic acid-producing bacteria are more active in the colon when GOS and pre-conditioned *L. reuteri* are added for 48 h.

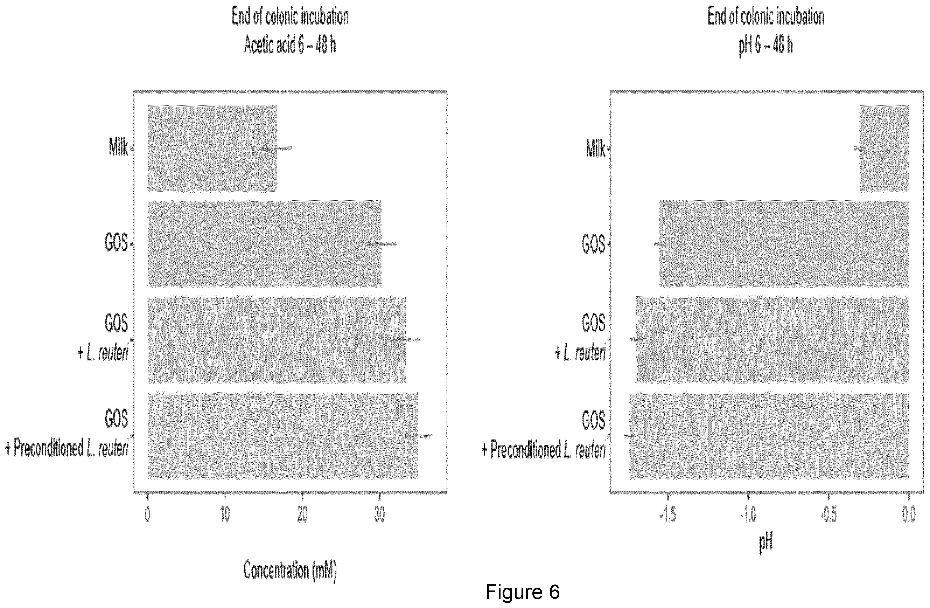

- **Figure 6:**: shows the pH decrease in the colon (difference between 48 h and 6 h) (right panel) and acetic acid production (difference between 48 h and 6 h) (left panel). GOS alone induces a pH drop and higher acetic acid production. *L. reuteri* added on top of GOS in the colon amplifies further the effect on pH and acetic acid production, meaning pH drops even more when *L. reuteri* is added to GOS. Pre-conditioned *L. reuteri* combined with GOS induces a stronger pH decrease in the colon. Higher level of acetic acid is also produced in presence of pre-conditioned *L*. reuteri and GOS, compared to GOS alone and GOS plus *L. reuteri.* pH is a marker of acid production. Both lactic acid and acetic acid are strongly induced by pre-conditioned *L. reuteri* in presence of GOS.

### DETAILED DESCRIPTION

The invention herein provides a way of enhancing probiotic effects of *Lactobacillus reuteri* strains in a mammal, using specific substrate components during fermentation when manufacturing the probiotic *L*. *reuteri* strains and further providing a surprisingly high synbiotic effect of the pre-conditioned probiotic *L. reuteri* strain and galacto-oligosaccharides (GOS) as further added in the final composition. The inventors of the present invention have thus developed a method which comprises the use of GOS during cultivation of the probiotic *L. reuteri* strain, so called pre-conditioning with GOS, and addition of GOS to the final composition, which surprisingly results in an unexpectedly high probiotic effect of the *L. reuteri* strain, particularly a significant increase of survival of the probiotic *L. reuteri* strain, an increased metabolic activity of the microbiome, bio-accessibility of minerals, e.g., by increasing mineral solubility, accessibility and/or absorption of minerals within the gastrointestinal tract of a subject or patient upon administering the pre-conditioned probiotic *L. reuteri* strain, as described herein. This beneficial and unexpected high effect is particularly generated due to the combined administration of additional GOS as a prebiotic and the (GOS-) pre-conditioned probiotic *L. reuteri* strain in one composition to a subject or patient in need thereof or in the form of a kit of parts.

The present invention therefore aims to provide compositions and uses, which increase mineral bio-accessibility and hence also mineral absorption in the gastrointestinal tract. In detail, such solutions are compositions comprising a GOS pre-conditioned *Lactobacillus reuteri* strain in combination with GOS further added in the final product to be administered to a subject in need of increased mineral uptake. The increased mineral solubility, accessibility, and absorption takes place in the gastrointestinal tract, such as in the duodenum, the jejunum, the ileum and/or in the colon.

The present invention also aims to provide methods for preparing a probiotic composition comprising a pre-conditioned probiotic *L. reuteri* strain in combination with GOS in the probiotic composition such that increased mineral solubility and bio-accessibility and also mineral absorption in the gastrointestinal tract can be achieved by virtue of the combination of such pre-conditioned *L. reuteri* strains in combination with GOS further added in the probiotic composition.

Objectives of the invention therefore relate to a method for preparing a probiotic composition comprising such a pre-conditioned probiotic *L. reuteri* strain in combination with additional GOS in the probiotic composition and, to the non-therapeutic and therapeutic use of a pre-conditioned probiotic L. *reuteri* strain in combination with GOS to increase mineral solubility, accessibility and/or absorption in the gastrointestinal tract of a mammal.

Additionally, an increased metabolic activity of the gut microbiome becomes evident e.g., through an increased lactic acid production. The observed increased lactic acid together with an increased acetic acid production indicate a higher activity of certain gut bacteria (including Lactobacillus and lactic acid producing bacteria) and shows that the microbiota has been beneficially modulated. The improved metabolic activity and improvement of mineral (iron and calcium) solubility of *L. reuteri* strain obtained by the combined administration of (GOS-) pre-conditioned probiotic *L. reuteri* and additional GOS, makes it possible to decrease the dosage and/or the frequency of administration of the probiotic and prebiotics, such as GOS, or to increase the beneficial effects of the same doses of conventional probiotics and/or prebiotics, required to obtain the sought after health effects.

In view of the above, the following is disclosed.

### METHODS FOR PREPARING A COMPOSITION COMPRISING PRE-CONDITIONED PROBIOTIC L. REUTERI BACTERIUM AND GOS

According to the first aspect the invention provides a method for preparing a probiotic composition, characterized in that the method comprises the steps:
a) preconditioning of a probiotic *Lactobacillus reuteri* strain following the steps:
   i) cultivating or culturing a probiotic *L. reuteri* strain in the presence of galacto-oligosaccharide (GOS) in a growth medium, thereby preconditioning the probiotic *L*. *reuteri* strain; and
   ii) optionally harvesting the pre-conditioned probiotic *L. reuteri* strain from the growth medium; and
b) preparing a composition comprising the pre-conditioned probiotic *L. reuteri* strain obtained from step a)i) or a)ii) and adding GOS to the composition.
The composition prepared according to the inventive method comprises the pre-conditioned probiotic *L*. *reuteri* strain and GOS.

In step a)i) of the inventive method, pre-conditioning of a probiotic *L. reuteri* strain occurs by cultivating a probiotic *L. reuteri* strain in the presence of GOS in a growth medium, thereby pre-conditioning the probiotic *L. reuteri* strain. In the context of the current invention, the term "pre-conditioning" of the probiotic *L. reuteri* bacteria preferably means growing, such as cultivating, culturing or fermenting, the probiotic *Lactobacillus reuteri* strain with GOS. In the inventive context, the terms "cultivation", "culturing" and "fermentation" are used interchangeably. Cultivation of a probiotic *L. reuteri* strain in the presence of GOS leads to formation of the pre-conditioned probiotic *L. reuteri* strain.

In optional step a)ii) the pre-conditioned probiotic *L. reuteri* strain is then harvested from the growth medium. The term "pre-conditioned *L. reuteri"* refers to a probiotic *L. reuteri* strain or strain produced by the inventive method including a pre-conditioning step with GOS. Harvesting step b) of such a GOS pre-conditioned probiotic *L. reuteri* strain is optional, as the GOS pre-conditioned probiotic *L. reuteri* strains may also be used directly in step b) for preparing a composition comprising the pre-conditioned probiotic *L. reuteri* strain obtained from step a)i) and adding GOS to the composition.

Furthermore, in step b) of the inventive method, the composition is formed by combining the pre-conditioned probiotic *L. reuteri* strain obtained from step a) and further adding GOS to the final composition.

A *L. reuteri* strain as used throughout the current invention may generally be any *L. reuteri* strain, more preferably any commercially available *L. reuteri* strain. In this context, the terms *"L. reuteri* strain" and *"L reuteri* bacteria" may be used synonymously. It is noted thereby that in the last decades DNA analysis tools have become more sophisticated, which has enabled scientists to discover many new bacterial species as well as realizing that the species historically grouped under the *Lactobacillus* genus were too diverse and did not conform to nomenclature conventions. To keep the probiotic groups accurate and organized, the genus *Lactobacillus* was therefore split into 25 different genera, including 23 novel genera. As a result, many probiotics have recently been given new genus names. Therefore, an alternative genus name for *Lactobacillus reuteri* (*L. reuteri*) is *Limosilactobacillus reuteri.* Since the reclassification process was very recent, we will use the former genera nomenclature, i.e., *Lactobacillus* for clarity purposes in this application. The revised genera nomenclature and alternative names should however be readily obtainable by anyone using available reclassification tools.

Currently preferred bacteria provided in the compositions are bacteria of at least one *L. reuteri* strain or of multiple *L. reuteri* strains, preferably as mentioned herein.

In a preferred embodiment of the current invention, *L. reuteri* strains as used herein is *L. reuteri* DSM 17938. *L. reuteri* DSM 17938 was deposited under the Budapest Treaty at the DSMZ-Deutsche Sammlung von Mikroorganismen und Zellkulturen GmbH (Mascheroder Weg 1b, D-38124 Braunschweig, Germany) on January 30, 2006.

Alternatively or additionally, the *L. reuteri* strain as used herein may be provided from at least one L. *reuteri* strain selected from *L. reuteri* as deposited under ATCC PTA 5289, *L. reuteri* strain ATCC PTA 6475, *L. reuteri* DSM 32846, *L. reuteri* DSM 32848, *L. reuteri* DSM 32849, *L. reuteri* DSM 27131, *L. reuteri* DSM 32465 or *L. reuteri* strain ATCC PTA 4659.

*Lactobacillus reuteri* DSM 27131 was deposited under the Budapest Treaty at the Leibniz Institut DSMZ-Deutsche Sammlung von Mikroorganismen und Zellkulturen GmbH (Inhoffenstr. 7B, D-38124 Braunschweig, Germany) on April 18, 2013.

*Lactobacillus reuteri* DSM 32846, DSM 32848 and DSM 32849 were deposited under the Budapest Treaty at the Leibniz Institut DSMZ-Deutsche Sammlung von Mikroorganismen und Zellkulturen GmbH (Inhoffenstr. 7B, D-38124 Braunschweig, Germany) on July 4, 2018.

*Lactobacillus reuteri* DSM 32465 was deposited under the Budapest Treaty at the Leibniz Institute DSMZ-German collection of Microorganisms and Cell Cultures (Inhoffenstr. 7B, D-38124 Braunschweig, Germany) on March 21, 2017.

*Lactobacillus reuteri* ATCC PTA 5289 was deposited under the Budapest Treaty at the American Type Culture Collection (10801 University Blvd., Manassas, VA 20110-2209, U.S.) on June 25, 2003.

*Lactobacillus reuteri* ATCC PTA 6475 was deposited under the Budapest Treaty at the American Type Culture Collection (10801 University Blvd., Manassas, VA 20110-2209, U.S.) on December 21, 2004.

*Lactobacillus reuteri* ATCC PTA 4659 was deposited under the Budapest Treaty at the American Type Culture Collection (10801 University Blvd., Manassas, VA 20110-2209, U.S.) on September 11, 2002.

The term "GOS" as used herein means "Galacto-oligosaccharide". Galacto-oligosaccharides (GOS) as used herein typically consist of β-linked galactose moieties with galactose or glucose at the reducing end. Such GOS contain β-(1→2), β-(1→3), β-(1→4), or β-(1→6) linked galactose moieties and may have a degree of polymerization (DP) of 3-8 galactose units. The term GOS is therefore preferably referred to as oligosaccharide(s) comprising at least three galactose units, more preferably as oligosaccharide(s) comprising at least four galactose units, preferably having a degree of polymerization (DP) of 3-8 galactose units. Oligosaccharides occur naturally in the milk of some mammals, e.g., cow and human. GOS are commercially available and can be synthesized via biosynthesis processes from lactose by trans-galactosidase activity of β-galactosidases. GOS formation in biosynthesis procedures is usually favored by high concentrations of lactose or lactulose, incomplete lactose turnover, low water activity, and the use of enzymes with preference for trans-galactosylation. The linkage type(s) of resulting GOS from such processes is(are) specific for the enzymes used for biosynthesis.

One particular type of mixture of carbohydrates that can advantageously be used herein as a source of GOS to grow the probiotic bacteria is a mixture of GOS and of cow's milk oligosaccharides. In particular mixtures of GOS with 3'-sialyllactose (3'-SL) and/or 6'-sialyllactose (6'-SL) are preferably used. Indeed, such mixtures contain some oligosaccharides similar to human milk, which is particularly advantageous when the composition of the invention is used as an infant formula or as a nutritional supplement for infants. Such advantageous effects are described for example in Simeoni et al.; "Gut microbiota analysis reveals a marked shift to bifidobacteria by a starter infant formula containing a synbiotic of bovine milk-derived oligosaccharides and Bifidobacterium animalis subsp. lactis CNCM I-3446"; Environ Microbiol, 2016, 18(7): 2185-2195. Such compositions can typically be obtained from concentrating whey permeate to obtain a concentrated bovine milk oligosaccharide composition and either adding GOS or generating the GOS in situ from hydrolysis of lactose by the action of a β-galactosidase.

The GOS source used in step a)i) of the inventive method to pre-condition the probiotic *L. reuteri* strain (*L. reuteri*) and the GOS source further added in step b) for preparing a final composition comprising the pre-conditioned probiotic *L. reuteri* strain with GOS, can be provided in the form of essentially pure GOS (i.e. ingredient having at least 90% GOS) or as part of a mixture, such as a mixture of carbohydrates, comprising GOS.

One aspect for the pre-conditioning of a probiotic *L. reuteri* strain with GOS is that a sufficient amount of GOS is provided in the growth medium. According to one embodiment the GOS source is therefore typically added to the growth medium in step a)i) of the inventive method for preparing a probiotic composition in an amount such as providing at least 0.2 wt%, preferably at least 0.5 wt%, even more preferably at least 0.75 wt% of GOS in the growth medium. In a preferred embodiment, GOS is provided in an amount such as providing at most 8 wt% of GOS, such as about 7 wt% GOS, 6 wt% GOS, or even 5 wt% GOS at most, more preferably at most 4 wt% of GOS or even 3 wt% GOS, in the growth medium, preferably calculated on basis of the growth medium (% w/w). Any combination of these upper and lower ranges is encompassed herewith. Preferably, GOS is added to the growth medium in a range of 0.2 wt% to 8 wt% or even 0.2 wt% to 7 wt%, or 0.2 wt% to 6 wt%, more preferably in a range of 0.5 wt% to 8 wt% or even 0.5 wt% to 7 wt%, or 0.5 wt% to 6 wt%, even more preferably in a range of 0.75 wt% to 8 wt% or even 0.75 wt% to 7 wt%, or 0.75 wt% to 6 wt%, and most preferably in a range of 0.75 wt% to 7 wt%, in a range of 0.75 wt% to 6 wt% or even in a range of 0.75 wt% to 5 wt%, such as a range of 0.75 wt% to 3 wt% or 0.75 wt% to 4 wt%, preferably calculated on basis of the growth medium.

When a mixture of carbohydrates is used as a source of GOS for pre-conditioning a probiotic *L. reuteri* strain or when preparing the final composition containing the pre-conditioned probiotic *L. reuteri* strain with GOS, it is preferred that the amount of GOS in such a mixture is at least 20 wt%, preferably at least 30 wt%, more preferably at least 40 wt%, even more preferably at least 45 wt%, most preferably at least 48 wt%, preferably calculated on a dry weight basis of the mixture containing GOS.

Although it is not necessary that the bacteria consume only GOS as carbon source during the fermentation, high proportion of GOS in the GOS source during the pre-conditioning step favors the consumption of GOS by the bacteria during fermentation over consumption of other carbohydrates, leading to improved pre-conditioning effect. Smaller carbohydrates such as di-saccharides (lactose for instance) can also be present and be consumed by the bacteria supporting the pre-conditioning effect. Accordingly, it is also preferred that during the pre-conditioning step a)i) the GOS source comprises at most 55 wt%, preferably at most 50 wt%, more preferably at most 45 wt%, most preferably at most 42 wt% of mono- or di-saccharides and/or no more than 40 wt%, preferably no more than 30 wt% lactose, preferably calculated on a dry weight basis of the mixture containing GOS. As an example, a mixture of carbohydrate used as a source of GOS during the pre-conditioning step may comprise GOS in amounts defined above for the mixture and the remainder formed by lactose, glucose, and/or galactose, and optionally 3'-SL and/or 6'-SL.

In an embodiment, the culture medium also comprises an electron acceptor, such as fructose, citrate, glycerol and/or 1,2-propanediol.

In a particular embodiment, the culture medium also comprises fructose, such as acting as an electron acceptor. In illustrative, but non-limiting, examples the culture medium may comprise at least 0.2 wt% fructose, preferably at least 0.5 wt% fructose, more preferably at least 1 wt% fructose, even more preferably at least 1.5 wt% fructose calculated on a dry weight basis of the culture medium. Correspondingly, in illustrative, but non-limiting, examples the culture medium preferably does not comprise more than 16 wt% fructose, preferably no more than 14 wt% fructose, more preferably no more than 12 wt% fructose and even more preferably no more than 10 wt% fructose. Any combination of these upper and lower ranges is encompassed herewith. In an embodiment, the culture medium comprises a wt% ratio between the electron acceptor (e.g. fructose) and GOS selected within a range of from 0.25:1 to 5:1, preferably within a range of from 0.5:1 to 4:1, and more preferably within a range of from 0.75 to 2.5:1. Particularly, preferred wt% ratios between the electron acceptor (e.g. fructose) and GOS are within a range of from about 1:1 to about 2:1.

According to a further embodiment, addition of GOS in step a)i) of the method for preparing a probiotic composition to the growth medium may occur at any point of time of the cultivation step a)i), e.g., at the start of cultivation of probiotic *L. reuteri* strain, in the middle or at the end of cultivation of probiotic *L*. *reuteri* strain in step a)i), wherein addition may occur either at once, stepwise or continuously.

The cultivation step a)i) is carried out in a way that is well known to the person skilled in the art. Cultivation in step a)i) includes the steps of inoculation of sterile standard growth medium with a defined amount of bacteria (cfu), followed by incubation under defined temperature (usually 37°C) and pH. In this context, a defined amount of bacteria (cfu) for starting an inoculation of sterile standard growth medium may be determined by a skilled person according to common general knowledge and practice in the art. Suitable yields can be obtained with the growth medium comprising GOS as described above, preferably without changing the cultivation conditions compared to what the person skilled in the art would use for cultivation of the same strain with a standard growth medium.

A standard growth medium for cultivating/fermenting the *L. reuteri* strain in step a)i) of the current invention may be any known standard growth medium used for lactobacilli and lactic acid bacteria (LAB), such as MRS medium, or any further suitable medium. Such a standard growth medium is preferably commercially available but may be also produced by addition of compounds according to well-known standard recipes. A standard growth medium for the *L. reuteri* strain may typically contain carbohydrates, such as simple sugars selected e.g., from dextrose, sucrose, maltose, fructose or lactose, various nitrogen sources, such as peptone, yeast extract, beef extract, or whey protein, minerals, mainly Mn²⁺ and Mg²⁺, and buffering agents, such as sodium acetate (CH₃COONa), trisodium citrate (Na₃C₆H₅O₇), or Di-sodium-glycerophosphate (C₃H₇Na₂O₆P) are commonly used buffers in LAB media. Other components that have been used in standard growth media with buffering activity may include disodium phosphate (Na₂HPO₄), ammonium citrate (NH₄C₆H₅O₇), trisodium phosphate (Na₃PO₄), potassium biphosphate (KH₂PO₄), magnesium phosphate tribasic Mg₃(PO₄)₂, calcium carbonate (CaCO₃), and dipotassium phosphate (K₂HPO₄). Such standard growth media may also contain a wide range of growth factors, surfactants, such as lecithin or Tweens (e.g., Tween 20, 80 and 85). The cultivation/fermentation may be carried out under anaerobic or aerobic conditions, depending on the strain to be produced, but preferably under anaerobic conditions. Also, the pH may be controlled or not, depending on the conditions known to be the best for a specific strain to grow. The temperature and duration of the cultivation step is variable from one strain to another and is also well-known to the person skilled in the art of probiotic the *L. reuteri* strain cultivation.

According to one embodiment the cultivation/fermentation of the probiotic *L. reuteri* strain in step a)i) of the inventive method occurs over a considerable period of time to allow a pre-conditioning of the probiotic *L. reuteri* strain in the presence of GOS, typically for a period of at least 1 hour, preferably for a period of between 5 to 18 hours, likewise preferably 7 to 14 hours, more preferably for a period of between 10 to 13 hours, and even more preferably for a period of about 12 hours.

Moreover, according to a further embodiment the cultivation/fermentation of the probiotic *L. reuteri* strain in step a)i) of the inventive method occurs at a temperature of between 25 to 45°C, preferably between 30 and 40°C, and even more preferably at a temperature of between 35 and 39°C, such as about 37°C.

Additionally, in another embodiment the cultivation/fermentation of the probiotic *L. reuteri* strain in step a)i) of the inventive method occurs at a pH of between 3.0 and 7. The pH during pre-conditioning of probiotic *L. reuteri* strain during cultivation step a) may be either not controlled or set to a constant value throughout the cultivation step a)i), typically to be monitored and adjusted during cultivation step a). Accordingly, the pH may be between 3.0 and 7, particularly when pH is not controlled during pre-conditioning of probiotic *L. reuteri* strain. Alternatively, if pH is controlled during cultivation step a)i), pH may be adjusted to a range of about 5.0 to 7.0, e.g., about 5.0 to 6.0, about 5.5 to 6.5 (e.g., about 6.0±0.2) or about 6.0 to 7.0 (e.g., about 6.6±0.2), more preferably pH may be adjusted to a range of about 5.5 to 6.5, even more preferably pH may be adjusted to a range of about 6.0±0.4, and most preferably pH may be adjusted to a range of about 6.0±0.2, likewise preferably pH may be adjusted to a range of about 6.0 to 7.0, likewise more preferably about 6.6±0.4, most preferably 6.6±0.2.

After cultivation/fermentation of a probiotic *L. reuteri* strain in step a)i) of the inventive method the thereby pre-conditioned probiotic *L. reuteri* strain is harvested in step a)ii). The harvesting step, which aims at separating the bacterial cells from the growth medium, is also carried out in a way that is well known to the person skilled in the art, such as by concentrating the bacteria, centrifugation, filtration, membrane-filtration, decantation, isolation, purification, etc., preferably centrifuging or concentrating the probiotic *L. reuteri* strain. Typically, harvesting of the pre-conditioned probiotic *L. reuteri* strain occurs in step a)ii) after a period of at least 1 hour as defined above for cultivation/fermentation in step a)i).

After harvesting the pre-conditioned probiotic *L. reuteri* strain in step a)ii) the harvested cells may be washed in an optional step a)iii) prior to further processing, typically to remove traces of growth medium (after cultivation/fermentation). Washing may occur with either saline water, brine, or any further suitable liquid.

Either directly after harvesting the pre-conditioned probiotic *L. reuteri* strain from the growth medium according to step a)ii) or after an optional washing step a)iii) the pre-conditioned probiotic *L. reuteri* strain may be subjected to a drying step a)iv). Such a drying step a)iv) may be carried out by any method known to a skilled person, such as spray-drying, fluid bed drying, air convective drying, atmospheric drying, roller drying or freeze drying, lyophilizing, and more preferably spray-drying or freeze drying. Spray drying may also be carried out in the presence of protecting agents, e.g., as described in WO 2017/001590.

Optionally, the pre-conditioned probiotic *L. reuteri* strain obtained after harvesting from the growth medium and/or after washing the pre-conditioned probiotic *L. reuteri* strain may further be mixed with protective agents, such as protectants, cryoprotectants, lyoprotectants and/or carriers before the drying step, as known to the person skilled in the art and as appropriate depending on the drying method to be used and the bacteria to be dried. Such protectants, cryoprotectants, lyoprotectants and/or carriers typically include glycerol, skimmed milk, serum albumin, peptone, yeast extract, saccharose, glucose, sorbitol, malt extract, trehalose etc. Commercially available protectants or cryoprotectants include e.g., 'Unipectine^{™} RS 150, etc. or are as described in EP 3016511, US 2014/0004083 A1, US 2016/0298077 A1, etc.

Alternatively, or additionally, the pre-conditioned probiotic *L. reuteri* strain obtained after culturing, after harvesting from the growth medium or after washing or even after drying, i.e. obtained after any of steps a)ii) or a)iii) after a)iv), may be encapsulated for further preservation and/or use, e.g. by formation of microspheres containing the pre-conditioned probiotic *L. reuteri* strain using common encapsulation agents, e.g. alginate, alginate/pullulan, starch, xanthan, xanthan-gellan gum mixtures, gelatin, cellulose acetate phethalate, chitosan, or any further suitable encapsulation material. Encapsulation is well known and can be carried out by a person skilled in the art.

Furthermore, the inventive method for preparing a probiotic comprises the step b) of preparing a composition comprising the pre-conditioned probiotic *L. reuteri* strain obtained from step a) and adding GOS to the composition. GOS may be applied in amounts as defined above, either pure or as a mixture as defined before.

According to a preferred embodiment, GOS may be added in step b) of the inventive method to the pre-conditioned probiotic *L. reuteri* strain obtained from step a) in an amount of at least 1 or 2 g per daily dose, preferably at least 3 g per daily dose, likewise preferably between 2 to 12 g per daily dose, or between 2 to 10 g per daily dose, between 2 to 8 g per daily dose, or between 2 to 7 g per daily dose, more preferably between 3 to 12 g per daily dose, likewise more preferably between 3 and 10 g per daily dose, such as between 3 to 8 g per daily dose, between 3 to 7 g per daily dose, between 3 to 6 g per daily dose, or about 2, 3, 4, 5, 6, 7, 8, 9, 10, 11, or 12 g per daily dose or any range formed thereby, to prepare the (final) composition. The GOS added to the composition comprising the pre-conditioned probiotic *L. reuteri* strain prepared in step b) has a degree of polymerization (DP) of 3-8.

### COMPOSITIONS COMPRISING THE PRE-CONDITIONED PROBIOTIC L. REUTERI BACTERIUM AND GOS

According to the second aspect, the invention provides a composition comprising an effective amount of a pre-conditioned probiotic *L. reuteri* strain and additionally an effective amount of a GOS, wherein the composition has been prepared/obtained according to the inventive method for preparing a probiotic composition. In this context, a pre-conditioned probiotic *L. reuteri* strain is a probiotic *L. reuteri* strain that has been cultivated in the presence of GOS, e.g. under conditions as defined above for the inventive method for preparing a probiotic composition. Such a pre-conditioning of a probiotic *L. reuteri* strain in the presence of GOS is typically carried out according to the inventive method as described herein under the first aspect of the invention and also as defined in the claims as attached. As already disclosed above in detail, cultivating/fermentation of a probiotic *L. reuteri* strain in the presence of GOS preferably occurs over a time sufficient to pre-condition a probiotic *L. reuteri* strain accordingly, e.g., over a period of at least one hour, preferably according to a process as defined herein.

As before, the *L. reuteri* strain is particularly preferably at least one *L. reuteri* strain or multiple *L. reuteri* strains as defined above, preferably selected from the group comprising or consisting of *L. reuteri* strain as deposited under DSM 17938, *L. reuteri* strain as deposited under ATCC PTA 5289, *L. reuteri* strain as deposited under ATCC PTA 6475, *L. reuteri* as deposited under DSM 32846, *L. reuteri* as deposited under DSM 32848, *L. reuteri* as deposited under DSM 32849, *L. reuteri* as deposited under DSM 27131, *L. reuteri* as deposited under DSM 32465 and *L. reuteri* strain as deposited under ATCC PTA 4659, more preferably the probiotic *L. reuteri* strain is *L. reuteri* strain as deposited under DSM 17938.

In one embodiment, the *L. reuteri* strain is particularly preferably at least one *L. reuteri* strain or multiple *L. reuteri* strains as defined above, preferably selected from group comprising or consisting *of L. reuteri* strain as deposited under DSM 17938, *L. reuteri* strain as deposited under ATCC PTA 5289, *L. reuteri* strain as deposited under ATCC PTA 6475, and *L. reuteri* strain as deposited under ATCC PTA 4659.

In one embodiment, the *L. reuteri* strain is particularly preferably at least one *L. reuteri* strain or multiple *L. reuteri* strains as defined above, preferably selected from group comprising or consisting *of L. reuteri* as deposited under DSM 32846, *L. reuteri* as deposited under DSM 32848, *L. reuteri* as deposited under DSM 32849, *L. reuteri* as deposited under DSM 27131, and *L. reuteri* as deposited under DSM 32465.

Typically, an "effective amount" of a pre-conditioned probiotic *L. reuteri* strain as defined herein for the composition may comprise a pre-conditioned probiotic *L. reuteri* strain typically in an amount of between 10³ cfu to 10¹² cfu, typically in an amount of between 10⁴ cfu to 10¹¹ cfu per daily dose, preferably in an amount of between 10⁵ cfu to 10¹⁰ cfu per daily dose or 10⁵ cfu to 10⁹ cfu per daily dose, likewise preferably in an amount of between 10⁶ cfu to 10⁹ cfu per daily dose, 10⁶ cfu to 10⁸ cfu per daily dose or in an amount of 10⁸ cfu to 10¹⁰ cfu per daily dose. A preferred daily dose is around 10⁸ total cfu's per daily dose, e.g., 10⁷ to 10⁹ or 10⁸ to 10⁹ cfu per daily dose.

Moreover, an "effective amount" of a GOS as defined herein for the composition may comprise GOS in an amount of at least 1 or 2 g per daily dose, preferably at least 3 g per daily dose, likewise preferably between 2 to 12 g per daily dose, or between 2 to 10 g per daily dose, between 2 to 8 g per daily dose, or between 2 to 7 g per daily dose, more preferably between 3 to 12 g per daily dose, likewise more preferably between 3 and 10 g per daily dose, such as between 3 to 8 g per daily dose, between 3 to 7 g per daily dose, between 3 to 6 g per daily dose, or about 2, 3, 4, 5, 6, 7, 8, 9, 10, 11, or 12 g per daily dose or any range formed thereby. The addition of GOS to the pre-conditioned probiotic *L. reuteri* strain as defined herein provides further improved synbiotic properties to the final product.

According to a particular embodiment, the inventive composition is for improving the probiotic effect, preferably of a *L. reuteri* strain. Such an improvement or increase of the probiotic effect may be:
- an increase of mineral bio-accessibility, preferably an increase of mineral solubility and/or mineral absorption, in the intestinal tract of a subject; and/or
- a promotion of a healthy microbiota in the intestinal tract of the subject; and/or
- a promotion of the survival of the *L. reuteri* strain in the intestinal tract (e.g. the colon) of the subject and thus a promotion of the health benefits associated to the *L. reuteri* strain; and/or
- an increase of the metabolic activity of the microbiota, e.g. through an increase in lactic acid/acetic acid production in the microbiota of a subject.

The inventive composition comprising an effective amount of a pre-conditioned probiotic *L. reuteri* strain and additionally an effective amount of a GOS, as defined herein may be any type of composition in which probiotic bacteria can be incorporated, such as a food product, a beverage, an animal feed product, a nutritional supplement for human or animal, a pharmaceutical composition or a cosmetic composition. More preferably, the inventive composition may be provided in an administrable form, preferably selected from the group consisting of food products, beverages, pharmaceutical formulations, dietary or nutritional supplements, functional food products, functional beverage products, nutraceuticals, and combinations thereof.

According to one particular embodiment may contain the effective amount of a pre-conditioned probiotic *L. reuteri* strain and additionally the effective amount of a GOS in two different parts of a kit-of-parts. The method or preparation as described before is then carried out as described before with the mere difference that the effective amount GOS is added toa different part of the kit-of-parts than the effective amount of a pre-conditioned probiotic *L. reuteri* strain.

The inventive composition may be solid or liquid. Preferably it is present in form of a powder, a tablet, a capsule, or may be in form of an oil formulation, an emulsion, e.g., an oil-in-water emulsion (o/w emulsion), a water-in oil emulsion (w/o emulsion), etc. If present in powder form it can be intended to be used by the final consumer in solid (such as powder form) or semi-solid form (such as for example in the form of a paste) or, alternatively, to be reconstituted into a liquid before use.

Food products and beverages as defined herein may include all products intended to be consumed orally by human beings, for the purpose of providing nutrition and/or pleasure. The expression "food product" as well as the term "beverages" usually mean compositions which nourish a subject. This "food product" is usually to be taken orally or intraperitoneally, and it can include a lipid or fat source and a protein source. Likewise, a "beverage" is usually to be taken orally, is liquid or a semi-liquid, and can include a lipid or fat source and a protein source.

Food products and beverages as defined herein can for example include a nutritional composition, preferably for human consumption, such as for infants and/or young children, for a pregnant or lactating woman or a woman desiring to get pregnant, for individuals in need of a special nutrition due to an adverse health condition or for elderly people. More preferably, the nutritional composition is selected from infant formula, infant cereals, follow-up or follow-on formula, growing-up milks, functional milks, baby food, infant cereal compositions, and milk products for pregnant and lactating women or for women desiring to get pregnant. Other examples of food products and beverages include sweet and savory snacks, powdered drinks, cereal products and dairy products, such as milk products, whey protein-based products, etc. According to one particular embodiment, the inventive composition is an infant formula, a follow-on formula, a growing-up milk or a product for pregnant or lactating women. In one further particular embodiment the inventive composition may be an infant formula.

The inventive composition can also be in the form of an animal food product or a nutritional supplement for animals. Preferably, the animal is a mammal. Examples of animals include, cows, sheep, goats, horses, dogs, cats, rabbits, rats, mice, fish, birds and the like.

The expression "infant formula" as used herein refers to a foodstuff intended for particular nutritional use by infants during the first months of life and satisfying by itself the nutritional requirements of this category of person (reference : Article 2(c) of the European Commission Directive 91/321/EEC 2006/141/EC of 22 December 2006 on infant formulae and follow-on formulae; Reg 609/2013 art 2.1.c and Reg 2016/127). It also refers to a nutritional composition intended for infants and as defined in Codex Alimentarius (Codex STAN 72-1981) and Infant Specialities (incl. Food for Special Medical Purpose). The expression "infant formula" encompasses both "starter infant formula" and "follow-up formula" or "follow-on formula", and includes compositions to be provided to premature infants.

A "follow-up formula" or "follow-on formula" is an infant nutritional composition given from the 6th month onwards. It constitutes the principal liquid element in the progressively diversified diet of this category of person.

The expression "baby food" means a foodstuff intended for particular nutritional use by infants or young children during the first years of life.

In the inventive context, the term "infant" means a child under the age of 12 months. Moreover, the expression "young child" means a child aged between one and three years, also called toddler.

The expression "infant cereal composition" means a foodstuff intended for particular nutritional use by infants or young children during the first years of life.

The product can also be in the form of an animal food product or a nutritional supplement for animals. Preferably, the animal is a mammal. Examples of animals include, cows, sheep, goats, horses, dogs, cats, rabbits, rats, mice, fish, birds and the like.

Dietary or nutritional supplements are typically present in the form of a liquid, such as a refrigerated liquid, in form of a powder or a tablet or capsule, an oil formulation, an emulsion, e.g., an oil-in-water emulsion (o/w emulsion), a water-in oil emulsion (w/o emulsion), etc. as mentioned above. Preferably it is in the form of a powder, a tablet, a capsule or an oil formulation. Powder supplements typically encompass supplements to be dissolved in a liquid or to be sprinkled on food or in a beverage. Such supplements are intended to provide additional nutrients and/or a health benefit to the subject consuming it, as well as other beneficial ingredients, such as the herein-defined and prepared pre-conditioned probiotic bacteria *L. reuteri* and additionally an effective amount of a GOS. A supplement according to the present invention can therefore be used for providing nutrients and/or a health benefit to human beings, as well as to animals, as defined above. Dietary or nutritional supplements include for example the herein-defined and prepared pre-conditioned probiotic bacteria *L. reuteri* and additionally an effective amount of a GOS as a powder supplement to be added to any sort of dietary or nutritional composition.

Pharmaceutical products include powder, tablet or capsule products intended to treat or prevent an adverse medical condition in a subject in need thereof, or to promote a favorable health condition.

Cosmetic compositions are typically intended for an aesthetic effect on the body and may be for topical use or may be administered by oral route, in the form of a powder, tablet or capsule.

### APPLICATIONS OF INVENTIVE COMPOSITIONS COMPRISING PRE-CONDITIONED PROBIOTIC L. REUTERI BACTERIUM AND GOS

The present invention aims to provide solutions, compositions and uses, which increase mineral solubility and bio-accessibility and also mineral absorption in the gastrointestinal tract. In detail, such solutions are compositions comprising an effective amount of GOS pre-conditioned probiotic *L. reuteri* strain and additionally an effective amount of a GOS to be administered to a subject or a patient in need thereof, e.g., a subject or a patient in need of increased mineral uptake. The increased mineral solubility, accessibility, and absorption takes place in the gastrointestinal tract, such as in the duodenum (first part of small intestine), the jejunum (middle part of small intestine), ileum and/or in the colon (large intestine). The present invention also aims to provide methods for preparing compositions comprising a pre-conditioned probiotic *L. reuteri* strain and additionally an effective amount of a GOS such that increased mineral solubility and bio-accessibility and also increased mineral absorption in the gastrointestinal tract can be achieved by virtue of such a combination.

As already outlined above, minerals are essential for all living species, even though the specific requirements differ between species. Mineral deficiencies can lead to disease and so can mineral excess. Correct amounts of minerals at the correct ratios are thus required for optimal health. Most natural diets will provide minerals in appropriate balances, but there are situations when a balanced diet is not enough to maintain health with respect to minerals. As mentioned, minerals are essential and have a great number of important functions in an organism.

Iron, for example, is responsible for the transport of oxygen and myoglobin. Moreover, iron is part of many enzymes such as catalases, peroxidases and cytochromes. Iron is generally absorbed in the duodenum and the upper part of the small intestine in the form of Fe²⁺ by cells that line the gastrointestinal tract, which absorbs iron from the ingested food.

Calcium is another essential mineral, which is inter alia an important bone component and an enzyme activator. Calcium also takes part in conduction of bioelectric impulses, blood coagulation, muscle contraction, inflammation and hormonal secretion. Calcium is generally absorbed in the small intestine in the presence of the active form of vitamin D (calcitriol).

A mineral deficiency of any of such minerals can lead to severe complications, conditions and diseases. The following uses, both non-therapeutic and therapeutic - are intended to address such mineral deficiency either by preventing or treating mineral deficiencies, e.g., in healthy patients in a non-therapeutic manner, e.g., by providing the inventive compositions as nutritional supplements or beverage, or in a patient in need thereof by administering or supplying the composition as defined above, e.g., as nutritional composition or beverage, as a pharmaceutical composition, etc.

The present invention also provides methods, solutions, compositions and uses which increase mineral solubility and bio-accessibility and also mineral absorption in the gastrointestinal tract using a probiotic composition comprising GOS pre-conditioned probiotic *L. reuteri* strain in combination with an additional amount of GOS, typically added to the final composition. Any of the herein described applications may be carried out with the inventive composition as described above or a kit-of-parts as described herein.

As shown herein inventive compositions allow for enhancing probiotic effects of *L. reuteri* strains in a mammal, using GOS as specific substrate components during fermentation when manufacturing the probiotic *L. reuteri* strains and supplementing such pre-conditioned probiotic *L. reuteri* strain further with GOS in the final composition. The herein disclosed method specifically provides for the preparation of such a composition, which pre-conditions in a first step the probiotic *L. reuteri* strain by use of GOS during cultivation of the probiotic *L. reuteri* strain, such that after addition of GOS in a second step a particularly increased beneficial probiotic effect of the *L. reuteri* strain occurs *in vivo,* particularly during non-therapeutic and therapeutic administrations as disclosed herein. This particularly concerns and allows e.g. an increase in survival of the probiotic *L*. *reuteri* strain, and an increase of bio-accessibility of minerals, e.g., by increasing mineral solubility, accessibility and/or absorption of minerals within the gastrointestinal tract of a subject or patient upon administering the composition comprising the pre-conditioned probiotic *L. reuteri* strain together with GOS, as described herein.

Additionally, an increased metabolic activity of the gut microbiome becomes evident e.g., through an increased lactic acid production upon administering the pre-conditioned probiotic *L. reuteri* strain supplemented with GOS, as described herein. The observed increased lactic acid together with an increased acetic acid production indicate a higher activity of certain gut bacteria (e.g. Lactobacillus and lactic acid producing bacteria) and shows that the microbiota has been modulated (resulting in a more healthy microbiota) due to administration of the pre-conditioned probiotic *L. reuteri* strain supplemented with GOS. The improved survival *of L. reuteri,* the significantly increased metabolic activity of colonic microbiome and the particular increase of mineral (iron and calcium) solubility in the colon can be attributed to the combined administration of the pre-conditioned *L*. *reuteri* further supplemented with GOS in the final composition. This combination conferred to a subject and/or patient in need thereof by administering the inventive composition makes it possible to decrease the dosage and/or the frequency of administration of the probiotic required to obtain the sought after health effects.
In the following, without being limiting thereto, exemplary therapeutic and non-therapeutic applications (uses) are listed.

### • NON-THERAPEUTIC USES OF COMPOSITIONS COMPRISING PRE-CONDITIONED PROBIOTIC L. REUTERI BACTERIUM AND GOS

According to the third aspect, the invention provides a non-therapeutic use of a composition comprising an effective amount of a pre-conditioned probiotic *L. reuteri* strain and an effective amount of GOS prepared according to the invention or as described herein, typically in form of an administrable composition, to increase or boost the probiotic effect of the *L. reuteri* strain in the digestive tract of a healthy subject. Such an increase or boosting of the probiotic effect by a pre-conditioned probiotic *L*. *reuteri* strain supplemented with GOS in the digestive tract of a healthy subject can be typically to be determined in comparison with the not yet pre-conditioned probiotic *L. reuteri* strain in the digestive tract of a healthy subject with or without supplementation with GOS.

The increase or boosting of the probiotic effect, typically of said probiotic *L. reuteri* strain in the digestive tract of a healthy subject, *inter alia* but not exclusively concerns the increase of the bio-accessibility of minerals, and specifically the increase of mineral solubility, bio-accessibility and/or absorption of minerals within the gastrointestinal tract of a subject. The increase of the bioavailability of minerals can in one embodiment result in an increased enzymatic function. Such minerals are preferably essential minerals as mentioned herein, e.g., calcium and/or iron. Increase of the probiotic effect may be manifested e.g., in improving the *L. reuteri* survival in the intestinal tract of a healthy subject, increasing lactic acid and/or acetic acid production in the intestinal tract of a subject, and/or promoting a healthy microbiota in a healthy subject.

According to a particular embodiment, a non-therapeutic use of a composition as defined herein is therefore preferably for improving or increasing the probiotic effect, preferably of a *L. reuteri* strain, in the digestive tract of a healthy subject. Such an improving or increasing the probiotic effect may be e.g.:
- an increase of mineral bio-accessibility, preferably an increase of mineral solubility and/or mineral absorption, in the intestinal tract of the healthy subject; and/or
- a promotion of a healthy microbiota in the intestinal tract of the healthy subject; and/or
- a promotion of the survival of the *L. reuteri* strain in the intestinal tract (e.g. in the colon) and thus a promotion of the health benefits associated to the *L. reuteri* strain; and/or
- an increase of the lactic acid/acetic acid production in the intestinal tract of the healthy subject; and/or
- an improvement of bone health, bone strength, bone formation, teeth conditions, teeth growth, muscular functions, cognitive functions, immune functions and/or general growth and development (especially for children/infants) due to an increase of mineral solubility and/or mineral absorption, in the intestinal tract of the healthy subject. The mineral may be selected from any of the herein mentioned minerals.

Of these essential minerals, calcium is involved in conduction of bioelectric impulses, blood coagulation, muscle contraction, prevention of inflammation and hormonal secretion. Iron is an important factor in many body functions and processes and is necessary to maintain healthy cells, skin, hair, and nails.

The increase of bio-accessibility of (such) minerals in the intestinal tract of the healthy subject, preferably by increasing mineral solubility, accessibility and/or absorption in the gastrointestinal tract of the healthy subject thereby preferably allows increasing any of such body functions and processes. These aspects particularly concern non-therapeutical and also cosmetical applications, for which the inventive compositions may be applied. It is emphasized that healthy subjects can clearly benefit from an improved availability of minerals by the claimed non-therapeutic uses as most minerals are not fully adsorbed by the body. In particular the healthy subjects can benefit from optimized or improved bone health, bone formation, teeth conditions, teeth growth, muscular functions, cognitive functions, immune functions and/or general growth and development (especially for children/infants).

In addition to such effects on mineral solubility, accessibility and/or absorption of minerals within the gastrointestinal tract of a subject further non-therapeutic uses may encompass an improvement of the *L. reuteri* strain metabolic activity and/or *L. reuteri* strain survival in the intestinal tract of a healthy subject, and/or promotion of a healthy microbiota in a healthy subject, and/or increase of the bio-accessibility of minerals in the intestinal tract of the healthy subject.

The inventors have evidenced a modulation or increase in lactic acid and acetic acid linked to the use of the composition of the invention. Lactic acid and acetic acid are strong anti-pathogenic molecules. Increasing their levels in the colon may increase the protection against pathogens and infections. Hence the composition according to the invention, in one embodiment, may promote defense against infections, especially gut infections and promote general immune function.

In this context, a healthy subject, which is the main addressee of this non-therapeutic use, may be selected from an athlete, a child, a toddler or an infant, an adult, an elderly, a pregnant woman, a vegetarian, a lactating woman, a companion animal, a cat, or a dog, or a bird, preferably for addressing or even improving such body functions and body processes. Such a healthy subject usually does not suffer from any condition or disease, particularly not from any mineral deficiency as described herein.

Any of the methods as discussed before for pre-conditioning a probiotic *L. reuteri* strain as well as any of the features of compositions as defined herein comprising the pre-conditioned probiotic *L. reuteri* strain supplemented with GOS apply *mutatis mutandis* for the third aspect of the current invention, namely to the non-therapeutic use of the inventive compositions as described above.

This specifically concerns features of the method of pre-conditioning a probiotic *L. reuteri* strain, the preparation of the composition with the pre-conditioned probiotic *L. reuteri* strain supplemented with GOS and any ingredients and amounts and further features as used for compositions comprising the pre-conditioned probiotic *L. reuteri* strain. The composition may be provided in any form as depicted above.

In this context, it is e.g., noted that the inventive composition for non-therapeutic purposes may be solid or liquid, and/or is present in form of a powder, a tablet, a capsule, or may be in form of an oil formulation, an emulsion, an oil-in-water emulsion (o/w emulsion), or a water-in oil emulsion (w/o emulsion).

Moreover, the inventive composition for non-therapeutic purposes may be in an administrable form, preferably selected from the group consisting of nutritional compositions, pharmaceutical formulations, dietary supplements, functional food products, functional beverage products, and combinations thereof, e.g., a dairy product, a milk-based product, a whey protein-based beverage.

As before, the *L. reuteri* strain is particularly preferable at least one *L. reuteri* strain or multiple *L. reuteri* strains selected from the group comprising or consisting of *L. reuteri* strain as deposited under DSM 17938, *L. reuteri* strain as deposited under ATCC PTA 5289, *L. reuteri* strain as deposited under ATCC PTA 6475, *L. reuteri* as deposited under DSM 32846, *L. reuteri* as deposited under DSM 32848, *L*. *reuteri* as deposited under DSM 32849, *L. reuteri* as deposited under DSM 27131, *L. reuteri* as deposited under DSM 32465 and *L. reuteri* strain as deposited under ATCC PTA 4659, preferably the probiotic *L. reuteri* strain is *L. reuteri* strain as deposited under DSM 17938.

### • USE OF COMPOSITIONS COMPRISING PRE-CONDITIONED PROBIOTIC L. REUTERI BACTERIUM AND GOS AS A MEDICAMENT

According to the fourth aspect, the invention also provides a composition comprising an effective amount of a pre-conditioned probiotic *L. reuteri* strain supplemented with GOS as prepared according to the invention or as described herein for use as a medicament.

Similar as before, the effect of such a composition as a medicament is particularly related to the increase of the synbiotic pre- and probiotic effects in the digestive tract of a patient in need of such a medicament, usually compared to the effect of a probiotic *L. reuteri* strain not pre-conditioned with GOS or usual combinations of pre- and probiotics.

The therapeutic effects that are related to the use of the inventive compositions as a medicament *inter* alia address effects related to any of the herein mentioned diseases and applications, particularly in patients that are at risk or that are already suffering from any of such diseases, particularly diseases as mentioned explicitly herein in the context of therapeutic applications (see also below).

Any of the methods as discussed before for pre-conditioning a probiotic *L. reuteri* strain as well as any of the features of compositions as defined herein comprising the pre-conditioned probiotic *L. reuteri* strain supplemented with GOS also apply *mutatis mutandis* for the fourth aspect of the current invention, namely to the use of the inventive compositions as a medicament.

This specifically concerns features of the method of preparing a composition comprising a pre-conditioned probiotic *L. reuteri* strain supplemented with GOS, and any ingredients and amounts and further features as used for the inventive compositions in any form as depicted above.

In this context, it is particularly preferred that the inventive composition for use as a medicament may be solid or liquid, may be present in form of a powder, a tablet, a capsule, or may be in form of an oil formulation, an emulsion, an oil-in-water emulsion (o/w emulsion), or a water-in oil emulsion (w/o emulsion).

Likewise, the inventive composition for use as a medicament may be in an administrable form, preferably selected from the group consisting of nutritional compositions, pharmaceutical formulations, dietary supplements, functional food products, functional beverage products, and combinations thereof, e.g., a dairy product, a milk-based product, a whey protein-based beverage.

As before, the *L. reuteri* strain is particularly preferable at least one *L. reuteri* strain or multiple *L. reuteri* strains selected from the group comprising or consisting of *L. reuteri* strain as deposited under DSM 17938, *L. reuteri* strain as deposited under ATCC PTA 5289, *L. reuteri* strain as deposited under ATCC PTA 6475, *L. reuteri* as deposited under DSM 32846, *L. reuteri* as deposited under DSM 32848, *L*. *reuteri* as deposited under DSM 32849, *L. reuteri* as deposited under DSM 27131, *L. reuteri* as deposited under DSM 32465 and *L. reuteri* strain as deposited under ATCC PTA 4659, preferably the probiotic *L. reuteri* strain is *L. reuteri* strain as deposited under DSM 17938.

### • THERAPEUTIC USES OF COMPOSITIONS COMPRISING PRE-CONDITIONED PROBIOTIC L. REUTERI BACTERIUM AND GOS

According to a fifth aspect, the invention provides a composition comprising an effective amount of a pre-conditioned probiotic *L. reuteri* strain supplemented with GOS as prepared according to the invention or as described herein for use in the prevention and/or treatment of a disease in a patient in need thereof or for reducing or inhibiting the risk of such a disease.

As used herein, the terms " treatment," "treat" and "to alleviate" include both prophylactic or preventive treatment (that prevent and/or slow the development of a targeted pathologic condition or disorder) and curative, therapeutic or disease-modifying treatment, including therapeutic measures that cure, slow down, lessen symptoms of, and/or halt progression of a diagnosed pathologic condition or disorder; and treatment of patients at risk of contracting a disease or suspected to have contracted a disease, as well as patients who are ill or have been diagnosed as suffering from a disease or medical condition. The term does not necessarily imply that a subject is treated until total recovery. The terms "treatment" and "treat" also refer to the maintenance and/or promotion of health in an individual not suffering from a disease but who may be susceptible to the development of an unhealthy condition, etc. The terms "treatment," "treat" and "to alleviate" are also intended to include the potentiation or otherwise enhancement of one or more primary prophylactic or therapeutic measure. The terms "treatment," "treat" and "to alleviate" are further intended to include the dietary management of a disease or condition or the dietary management for prophylaxis or prevention a disease or condition.

According to one embodiment, a disease in a patient in need thereof may concern e.g., mineral deficiency. Accordingly, a patient in need thereof is also typically a subject that is at risk of developing mineral deficiency or is a subject already suffering from mineral deficiency. Such a patient may be any patient group as described above herein. Such a patient in need thereof may also be a patient is at risk of suffering from a digestive dysfunction, an impaired microbiota, and/or has a digestive dysfunction, an impaired microbiota or a condition requiring promotion of the development and/or growth of the microbiota.

Mineral deficiency in this particular context of the invention preferably concerns a deficiency in calcium and/or iron. Thereby, it is not necessarily a deficiency of just one mineral alone that may lead to a specific disease as depicted below but more often a combined lack of different minerals, e.g., one or more minerals selected from calcium and/or iron, and, consequently, also an overlap in the physiological consequences and a combination of different diseases/conditions that may occur.

As mentioned before, iron deficiency is a condition, which occurs when the body doesn't have enough of the mineral iron. Iron deficiency often leads to abnormally low levels of red blood cells and a condition referred to as iron deficiency anemia. As a result, iron deficiency anemia results in tiredness and shortness of breath. Iron is also known to be very important in maintaining many body functions and is necessary to maintain healthy cells, skin, hair, and nails.

The following groups of people are at highest risk for iron-deficiency anemia and are hence suitable for receiving the GOS supplemented pre-conditioned *L. reuteri* strain composition; women who menstruate, particularly if menstrual periods are heavy, women who are pregnant or breastfeeding or those who have recently given birth, people who have undergone major surgery or physical trauma and lost blood, people with gastrointestinal diseases such as celiac disease, inflammatory bowel diseases such as ulcerative colitis, or Crohn's disease, people with peptic ulcer disease, people who have undergone bariatric procedures, especially gastric bypass operations, vegetarians, vegans, and other people whose diets do not include iron-rich foods, children who drink more than 16 to 24 ounces a day of cow's milk (Cow's milk not only contains little iron, but it can also decrease absorption of iron and irritate the intestinal lining causing chronic blood loss.), and infants especially those who have low birth weight or are born prematurely, and/or those who don't get enough iron from breast milk or formula. In general, children need extra iron during growth spurts. Any of such patient groups may be treated herein.

Thus, in one embodiment the inventive composition comprising an effective amount of the pre-conditioned probiotic *L. reuteri* strain is as prepared according to the invention or as described herein used to treat iron deficiency, preferably iron deficiency, preferably iron deficiency anemia, in a subject.

Further, chronic iron deficiency is a known emerging risk factor for bone loss and osteoporosis. Calcium is another essential mineral, which is important bone component and an activator of enzymes. Calcium also takes part in conduction of bioelectric impulses, blood coagulation, muscle contraction, inflammation and hormonal secretion.

In one embodiment, the present invention therefore provides a composition comprising a pre-conditioned *L. reuteri* strain for use in the prevention and/or treatment of calcium deficiency in a subject, for use in the prevention and/or the treatment of bone loss, or for use in the treatment of osteoporosis and/or osteopenia.

Moreover, and particularly in view of calcium deficiencies, further conditions and/or diseases may be prevented or treated related to muscle problems, e.g., cramps, muscle spasms, and aches, fatigue, including insomnia, sleepiness, and extreme fatigue, skin and nail symptoms, eczema, psoriasis, redness, itchiness, skin blisters, osteoporosis and osteopenia, painful premenstrual syndrome, dental problems, depression, etc. Moreover, calcium deficiency (hypocalcemia) is also serious problem in early infancy, which makes infants of said stage to a particular target group. This includes neonatal hypocalcemia, which occurs in the first 2 to 3 days of a baby's life, as well as late hypocalcemia, which starts in the first week or weeks after birth. Any of such diseases may be treated. According to one embodiment, the present invention particularly provides a composition comprising a pre-conditioned *L*. *reuteri* strain for use in the promotion of cognitive functions, promotion of oxygen transport, promotion of immune functions, and/or reduction of tiredness or fatigue, in a patient in need thereof.

According to a further embodiment the present invention provides a composition comprising a pre-conditioned probiotic *L. reuteri* strain supplemented with GOS for use in increasing the probiotic effect of a probiotic *L. reuteri* strain. Such an increase of the probiotic effect is preferably an increase of mineral bio-accessibility, more preferably an increase of mineral solubility and/or mineral absorption, in the intestinal tract of the patient; and/or is a promotion of a healthy microbiota in the intestinal tract of the patient, and/or is an increase of the lactic acid/acetic acid production in the microbiota of the patient. Any of such treatments may be addressed.

Such an increase of bio-accessibility of minerals in the intestinal/digestive tract of a subject is particularly due to the GOS pre-conditioning step of preparing the herein used a pre-conditioned *L*. *reuteri* strain and the observed increased capability of said pre-conditioned *L. reuteri* strain supplemented with GOS to positively influence and increase bio-accessibility of minerals in the intestinal tract of the healthy subject, typically by increasing mineral solubility, accessibility and/or absorption of such minerals in the gastrointestinal tract of a subject. Particularly relevant minerals in this context include calcium and/or iron.

An embodiment relates to a composition comprising an effective amount of a pre-conditioned probiotic *L. reuteri* strain and an effective amount of GOS as prepared according to the invention for use in the prevention and/or treatment of an impaired microbiota or a dysbiosis.

A further positive effect of the herein used pre-conditioned *L. reuteri* strain supplemented with GOS moreover is therefore also an improved microbiota and also an increase in the lactic acid/acetic acid production in the microbiota. It is therefore submitted that an increase in survival and/or engraftment and/or viability of a *L. reuteri* strain in the intestinal tract of a patient to be treated may specifically and positively influence the treatment of any of the herein-mentioned diseases. The medical uses as claimed herein therefore also encompass an increase in mineral solubility and/or mineral accessibility and/or mineral absorption in the digestive tract of a patient in need thereof, and/or an increase of the metabolic activity of the gut microbiota of a patient in need thereof, and/or an increase the lactic acid/acetic acid production in the microbiota of a patient in need thereof, typically in the context of any of therein mentioned conditions or diseases. The medical uses herein also concern the prevention and/or the treatment of bone loss, the promotion of promote growth, the development of bones or teeth in children, teeth growth, promotion of muscular functions, promotion of functions of the nervous system and/or bone or muscular related conditions, in a patient in need thereof. The medical uses herein also concern the prevention and/or the treatment of infections, preferably gut infections, promotion of immune functions, which is preferably enabled by the increase of lactic acid and/or acetic acid production in the microbiota of the patient. Any of such treatments may be addressed herein.

Additionally or alternatively, the promoted probiotic effect can encompass the promotion of the digestive functions, of the immune function and/or of the growth and development, especially in subjects suffered from impairment of their microbiota, and/or having a condition requiring such promotion. Such conditions may include e.g., any treatment or disease that diminishes the amount and/or function of (beneficial) bacteria in the microbiota, such as immunotherapies, cancer therapies, administration of antibiotics, patients suffering from diarrhea, from infections or inflammations, but may also and particularly concern any such and further conditions related to an impaired microbiota or a condition requiring promotion of the development and/or growth of the microbiota that affect infants and small children.

Generally, subject for any such treatments or a patient in need of such a treatment is a mammal or a bird, preferably, it is human, more preferably it is selected form a child, a toddler or an infant, an elderly, a pregnant woman, a vegetarian, a lactating woman, a companion animal, but also companion pets such as a cat or a dog, wherein the subject is preferably either at risk of developing mineral deficiency or has already developed a mineral deficiency. Populations at risk for calcium deficiency and preferred subject to such a prevention and/or treatment particularly include pregnant women (especially in the last trimester), lactating women, postmenopausal women, older adults, teenagers, and/or people who are overweight. Populations at risk for hypomagnesemia particularly include diabetes patients, patients at risk of diabetes, obese patients and/or overweight patients, etc. Any of such subjects and patient groups may be treated herein.

Also, in this context, any of the methods as discussed before for preparing a composition comprising a pre-conditioned probiotic *L. reuteri* strain supplemented with GOS as well as any of the features of compositions as defined herein comprising the pre-conditioned probiotic *L. reuteri* strain also apply *mutatis mutandis* for the fifth aspect of the current invention, namely to the medical or therapeutic uses of the inventive compositions in any of the herein defined treatments.

This specifically concerns features of the method of preparing a composition comprising a pre-conditioned probiotic *L. reuteri* strain supplemented with GOS, and any ingredients and amounts and further features as used for compositions comprising the pre-conditioned probiotic *L. reuteri* strain. The composition may be provided in any form as depicted above.

In this context, it is particularly preferred that the inventive composition for medical or therapeutic uses as described herein may be solid or liquid, may be present in form of a powder, a tablet, a capsule, or may be in form of an oil formulation, an emulsion, an oil-in-water emulsion (o/w emulsion), or a water-in oil emulsion (w/o emulsion).

Likewise, the inventive composition for medical or therapeutic uses as described herein may be in an administrable form, preferably selected from the group consisting of nutritional compositions, pharmaceutical formulations, dietary supplements, functional food products, functional beverage products, and combinations thereof, e.g., a dairy product, a milk-based product, a whey protein-based beverage.

As before, the *L. reuteri* strain is particularly preferable at least one *L. reuteri* strain or multiple *L. reuteri* strains selected from the group comprising or consisting of *L. reuteri* strain as deposited under DSM 17938, *L. reuteri* strain as deposited under ATCC PTA 5289, *L. reuteri* strain as deposited under ATCC PTA 6475, *L. reuteri* as deposited under DSM 32846, *L. reuteri* as deposited under DSM 32848, *L*. *reuteri* as deposited under DSM 32849, *L. reuteri* as deposited under DSM 27131, *L. reuteri* as deposited under DSM 32465 and *L. reuteri* strain as deposited under ATCC PTA 4659, preferably the probiotic *L. reuteri* strain is *L. reuteri* strain as deposited under DSM 17938.

According to a further particular embodiment, the current invention also provides a method of treatment of a disease in a patient in need thereof. Such a disease and a patient are preferably as described herein. The method of treatment preferably includes the step of administering to the patient an effective amount of a pre-conditioned probiotic *L. reuteri* strain, preferably as prepared according to the inventive method for pre-conditioning the probiotic *L. reuteri* strain, or as described herein. A pre-conditioned probiotic *L. reuteri* strain is thereby a probiotic *L. reuteri* strain that has been cultivated in the presence of GOS, preferably according to the inventive method for pre-conditioning the probiotic *L. reuteri* strain. Administration allows preferably the prevention and/or treatment of a disease in a patient in need thereof or reducing or inhibiting the risk of such a disease. Any of the diseases as defined above are encompassed. The effective amount of a pre-conditioned probiotic *L. reuteri* strain may be provided in form of an inventive composition as described herein. Any of the features discussed above for the inventive method for pre-conditioning the probiotic *L. reuteri* strain and the inventive composition analogously apply.

According to another particular embodiment, the current invention also provides the use of a pre-conditioned probiotic *L. reuteri* strain, preferably as prepared according to the inventive method for pre-conditioning the probiotic *L. reuteri* strain, or as described herein for preparing a medicament or pharmaceutical composition for preventing and/or treating a disease in a patient in need thereof. Such a disease and a patient are preferably as described herein. A pre-conditioned probiotic *L. reuteri* strain is thereby a probiotic *L. reuteri* strain that has been cultivated in the presence of GOS, preferably according to the inventive method for pre-conditioning the probiotic *L. reuteri* strain. The use preferably allows the prevention and/or treatment of a disease in a patient in need thereof or reducing or inhibiting the risk of such a disease. Any of the diseases as defined above are encompassed. The effective amount of a pre-conditioned probiotic *L. reuteri* strain may be provided in form of an inventive composition as described herein. Any of the features discussed above for the inventive method for pre-conditioning the probiotic *L. reuteri* strain and the inventive composition analogously apply.

It should be appreciated that the various aspects and embodiments of the detailed description as disclosed herein are illustrative of the specific ways to make and use the invention and do not limit the scope of invention when taken into consideration with the claims and the detailed description. It will also be appreciated that features from aspects and embodiments of the invention may be combined with further features from the same or different aspects and embodiments of the invention.

As used in this detailed description and the appended claims, the singular forms "a," "an" and "the" include plural referents unless the context clearly dictates otherwise.

All ranges described are intended to include all numbers, whole or fractions, contained within the said range.

### EXAMPLES

### Example 1 - Method for pre-conditioning L. reuteri with GOS

Internally produced *L*. *reuteri* frozen starter cultures (*L. reuteri* DSM 17938) were used to inoculate a fermentation medium containing 4% Bovine milk derived oligosaccharides (BMOS) as carbon source and 4% fructose as electron acceptor (on dry matter). Overnight culture *of L. reuteri* was used to inoculate a fresh fermentation medium containing 6% BMOS as carbon source and 6% Fructose as electron acceptor (on dry matter). Fermentation was run under non pH control and at 37°C. Pre-conditioned *L. reuteri* cells were harvested by centrifugation after around 10 h fermentation prior to mixing with protectants and to spray-drying.

BMOS carbohydrate mixture used as source of GOS was composed of 48% GOS, 30% lactose, 8% glucose, 3.9% galactose, and >0.2% 3'-SL +6'-SL (g/100g of dry matter).

*L. reuteri* cell counts were determined by pour plating. Briefly, serial decimal dilutions spray dried samples were performed in tryptone salt (Oxoid, LP0042) solution. Subsequently, 100 µL of the appropriate dilutions were transferred to petri dishes and mixed with MRS agar (AES Chemunex, Bruz, France). Analysis was performed in duplicate. Plates were then incubated in aerobic conditions at 37°C for 48 h. Colony forming units per gram (cfu/g) were calculated from the number of colonies counted on plates with appropriate dilution by determining their arithmetic mean.

Furthermore, according to an optional step, highly purified GOS (>93%) was added to the harvested pre-conditioned probiotic *L. reuteri.*

### Example 2 - Manufacture of a sachet comprising pre-conditioned L. reuteri and GOS

The composition is made of: lyophilized pre-conditioned *L. reuteri* DSM 17938 (cultivated and lyophilized as described below), 10⁸ CFU/sachet and GOS 15 (VIVINAL^{®}, FrieslandCampina Domo, The Netherlands) 2000 mg/sachet. The composition is filled at ambient temperature into aluminium foil bags as known in the art with desiccant (10 cm x 12 cm, using packaging material PET12/PE/ALU12/PE/PE-desiccant/PE from Alcan) in a LAF bench (Holten Laminair Model S-2010 1.2 from Heto-Holten A/S, Denmark). To each bag, 2 g of powder with *L. reuteri* and GOS is added using the balance XP-600 from Denver Instrument GmbH, Germany. The filled aluminum foil bags are then heat sealed with the film sealing device mode F460/2 from Kettenbaum Folienschweisstechnik GmbH & Co. KG, Germany.

### a) Cultivation in bioreactor

Ten ml MRS media (Oxoid) is inoculated with 100 µl of *L. reuteri* DSM 17938 from a frozen stock stored at -70°C. The tube is incubated for 6 h at 37 °C and thereafter 500 µl is used for inoculation of a tube with 50 ml MRS. The tube is incubated at 37 °C for 16-20 hours and thereafter the whole volume is used as inoculum to the bioreactor.

### Conditions for cultivation in bioreactor:

Working volume: 1 L
Medium: iMRS* with 3% BMOS+3% fructose
Aeration: No aeration
Temperature: 37 °C
Stirring: 250 rpm
pH: 6.00 +/- 0.1
Cultivation time: 6-10 h

### *iMRS ingredients

| Component | Quantity(g) |
|---|---|
| Yeast extract | 10 |
| Yeast peptone | 10 |
| Sodium citrate | 2 |
| Sodium acetate | 5 |
| K₂HPO₄ | 2 |
| MgSO₄ heptahydrate | 0.1 |
| Manganese sulfate heptahydrate | 0.05 |
| Tween 80 | 1 |
| L-cysteine | 0.1 |
| Purified water (800 ml) | |
| Set pH to 6,5 with NaOH | |

Autoclave (121°C 15 min) and thereafter add sterile sugar solution (final volume 1000 ml)

After cultivation, the bacteria are pelleted by centrifugation and suspended in 50 ml 10% sucrose.

### b) Lyophilization

The concentrated cell suspensions are dispensed into freeze-drying glass vials (1 ml/vial) and the vials are frozen in an ultra-freezer at -50 °C for 2 h before being transferred to a Labconco FreeZone Stoppering Tray Freeze-Dryer. The freeze-drying scheme is set-up as follows: Freezing step: -40 °C for 4h; Main drying: -35 °C for 15 min at 0.102 mbar, -10 °C for 12.5 h at 0.102 mbar; secondary drying: -10 °C for 30 min at 0.01 mbar, 25 °C for 12 h at 0.01 mbar. At the end of the process the vials were capped under vacuum and stored at -20 °C until further use.

### Example 3 - Method for preparing a probiotic composition comprising pre-conditioned L. reuteri supplemented with additional GOS

A cow milk-based toddler beverage, also called growing-up-milk, containing minerals adapted for the age group was prepared by adding an amount of 5x10⁶ cfu per daily dose pre-conditioned probiotic *L*. *reuteri* and an amount of GOS of 0.5 to 12 g per daily dose reconstituted growing-up-milk composition.

### Example 4 - Composition comprising pre-conditioned L. reuteri supplemented with additional GOS increases survival in colon and increases calcium and iron solubility in colon.

### a) SHIME-model

In this experiment, a simplified simulation of the continuous Simulator of the Human Microbial Ecosystem (SHIME^{®}) was used. This SHIME^{®} model has been extensively used for more than 20 years for both scientific and industrial projects and has been validated with in vivo parameters (see for example Van den Abbeele et al., Arabinoxylo-Oligosaccharides and Inulin Impact Inter-Individual Variation on Microbial Metabolism and Composition, Which Immunomodulates Human cells, J. Agric. Food chem. 2018, 66, 5, 1121-1130).

For the current set of experiments a two-stage batch system mimicking the upper gastro-intestinal tract (Upper GIT, stomach and small intestine) and colonic conditions were used as simplified SHIME^{®} system (Figure 1). Cow milk-based toddler beverage, also called Growing-up-milk, containing minerals adapted for the age group was used in these studies.

In order to simulate the absorptive processes occurring in the small intestine of toddlers, a dialysis approach was applied by using a cellulose membrane with a cut-off of 14 kDa. By introducing the small intestinal suspension within a dialysis membrane, molecules such as digested amino acids, sugars, micronutrients and minerals were gradually removed from the upper gastro-intestinal matrices.

Furthermore, a gradual pH decrease during the stomach incubation going from 5.5 till 3.0 during 1 h of incubation was implemented to simulate the gastric pH of toddlers. Also, during the first 30 minutes of small intestinal incubation (duodenum), a fixed pH of 4.5 was implemented to allow the available minerals to optimally absorb. The following 145 minutes of the small intestinal phase (jejunum + ileum), a pH of 7 was introduced. The milk matrix after exposure to gastric and small intestinal conditions was transferred to the colonic compartment containing the fecal sample of a toddler.

Fresh fecal material was collected from a 12-month-old infant donor. Fecal suspension was prepared and mixed with a protectant. At the start of the short-term colonic incubation, the test ingredients (*L. reuteri* strain and *Streptococcus thermophilus* strain) were added to sugar-depleted nutritional medium containing basal nutrients present in the colon (e.g., host-derived glycans such as mucin).

*L. reuteri* strain and S. *thermophilus* strain were inoculated at 1E+09 CFU/reactor.

Following incubations were performed:
- Control incubation without probiotic (Upper GIT-digested milk alone)
- Upper GIT digested milk + *L*. *reuteri* strain DSM 17938
- Upper GIT digested milk + S. *thermophilus* NCC2496
- Upper GIT digested milk + GOS + *L*. *reuteri* strain DSM 17938
- Upper GIT digested milk + GOS + S. *thermophilus* NCC2496

Highly purified GOS (>93% purity) was used at equivalent of 4 g GOS/L of digested milk to simulate GOS structures reaching the large intestine.

Internally produced S. *thermophilus* frozen starter cultures were used to inoculate a fermentation medium containing 3% dextrose as carbon source, 2.5% yeast extract as nitrogen source and 0.1% Tween 80. Overnight culture at 37°C of S. *thermophilus* was used to inoculate a fresh fermentation medium containing 4% dextrose as carbon source, 2.5% yeast extract as nitrogen source and 0.1 % Tween 80. Fermentation was run at 40°C and under pH control maintained at 5.8 with addition of NaOH 30%.

In a separate experiment, the test ingredients (*L. reuteri,* pre-conditioned *L. reuteri*) were added into the colonic medium containing the fecal sample. BMOS was replaced by dextrose (8%) in the fermentation medium *of L. reuteri* (reference=not pre-conditioned). Same fermentation conditions were then applied (fermentation time of 10 h, no pH control, 37°C).

*L. reuteri* strain and pre-conditioned *L. reuteri* strain were inoculated at 1E+09 CFU/reactor. *L. reuteri* were quantified by plating on MRS medium, while S. *thermophilus* was plated on M17 medium supplemented with lactose. Both strains were incubated at 37°C for 48h before cell counting. Quantities of *L. reuteri* and S. *thermophilus* powders were then calculated to target 1E+09 CFU/colonic reactor.

Following incubations were performed:
- Control incubation without probiotic (Digested milk alone)
- Upper GIT digested milk + GOS
- Upper GIT digested milk + *L*. *reuteri* strain DSM 17938
- Upper GIT digested milk + pre-conditioned *L. reuteri* DSM 17938
- Upper GIT digested milk + GOS + *L*. *reuteri* strain DSM 17938
- Upper GIT digested milk + GOS + pre-conditioned *L. reuteri* DSM 17938

Colonic incubations were performed during 48 h, at 37°C and under shaking (90 rpm) conditions. All experiments were performed in triplicate to account for biological variation.

### b) Analysis of minerals (calcium and iron):

Analysis of calcium and iron was performed by a specialized laboratory of Ghent University, Belgium. The samples were measured by ICP-OES. Samples for mineral analysis in the colon compartment were taken after 0 h, 6 h, 24 h and 48 h. The 48 h time point was preceded by a colonic dialysis, resulting in values for the bio-accessible mineral fraction and the insoluble mineral fraction. Samples taken at 0 h, 6 h and 24 h were centrifuged and filtered before analysis. Total calcium was measured before centrifugation. Soluble calcium was measured in the fraction after centrifugation and filtration (pellet excluded).

Results are shown in Figures 2 and 3.

As can be seen in Figure 2 GOS alone has little or no effect on calcium solubility. *L. reuteri* combined with GOS boost the solubility of calcium at both 6 h and 24 h. Pre-conditioned probiotic *L. reuteri* strain further boosts calcium solubility in colon at both 6 h and 24 h in presence of GOS. This suggests that a pre-conditioned *L. reuteri* strain combined with GOS can be used to promote calcium absorption in a subject in need thereof.

As can be seen in Figure 3, pre-conditioned probiotic *L. reuteri* strain in combination with GOS boosts iron solubility (bio-accessibility) in the colon suggesting that the combination of both GOS and pre-conditioned *L. reuteri* can promote iron absorption in a subject in need thereof.

### c) Microbial community composition

qPCR was performed to selectively enumerate Lactobacilli and Streptococci. qPCR was performed at 0 h and after 48 h of colonic incubation.

Results are shown in Figures 4A and 4B.

Figure 4A shows that survival of *L. reuteri* in the colon is enhanced by the presence of GOS. In contrast, survival of S. *thermophilus* is impaired by the presence of GOS in the colon. It means that even if both strains are able to grow in presence of GOS in fermentation medium and under controlled conditions, the strains behave differently when they are in a highly competitive environment like in the colon. Diverse bacteria are present in the gut, especially in the colon. Bacteria fight for food to survive in the colonic environment. *L. reuteri* is able to consume GOS in an efficient manner leading to a good survival. S. *thermophilus* seems to struggle more in competitive bacterial environment even though the strain has the ability to consume GOS.

Figure 4B shows the survival of pre-conditioned *L. reuteri* in the colon in presence of GOS. *L. reuteri* (no pre-conditioning) survives well in the colon in presence of GOS. Pre-conditioning improves the survival of *L. reuteri* as seen by higher cell counts at 48 h in the colon.

### d) Microbial metabolic activity

To assess the microbial activity of the pre-conditioned probiotic *L. reuteri* strain prepared according to the inventive method the pH, short chain fatty acids and lactate values were monitored over a period of 0 h, 6 h and 48 h after starting the incubation:
- -pH: The degree of acidification during the experiment is a measure for the intensity of bacterial metabolism (fermentation). The pH of the incubations was determined at 0 h, 6 h and 48 h after starting the incubation, thus giving a rough indication on the speed of fermentation.
- Short chain fatty acids (SCFA) are an assessment of the microbial carbohydrate metabolism (acetate in this case) can be compared to typical fermentation patterns for normal GI microbiota. Samples for SCFA analysis were analyzed after 0 h, 6 h and 48 h of incubation.
- Lactate: The intestine harbors both lactate-producing and lactate-consuming bacteria. Lactate is produced by lactic acid bacteria and decreases the pH of the environment, acting also as an antimicrobial agent. It can also be rapidly converted to acetate, butyrate, and propionate by other microorganisms. Samples for lactate analysis were analyzed after 0 h, 6 h, and 48 h of incubation.

Results are shown in Figures 5 and 6.

Figure 5 shows the lactic acid production (difference between the level at 48 h and 6 h). GOS alone induces a strong lactic acid production in the colon. Addition of *L. reuteri* in combination of GOS further enhances the production of lactic acid in the colon. The highest production of lactic acid was measured when pre-conditioned *L. reuteri* was added in combination of GOS in the colon, showing an overall increase of the metabolic activity of the microbiome. Lactic acid producing bacteria are more active in the colon when GOS and pre-conditioned *L. reuteri* are added for 48 h.

Figure 6 shows the pH decrease in the colon (difference between 48 h and 6 h) (right panel) and acetic acid production (difference between 48 h and 6 h) (left panel). Pre-conditioned *L. reuteri* combined with GOS induces a stronger pH decrease in the colon. Higher level of acetic acid is also produced in presence of pre-conditioned *L. reuteri* and GOS. pH is a marker of acid production. Both lactic acid and acetic acid are strongly induced by pre-conditioned *L. reuteri* in presence of GOS.

## Claims

1. A method for preparing a probiotic composition, **characterized in that** the method comprises the steps:
a) pre-conditioning of a probiotic *Lactobacillus reuteri* strain following the steps:
i) cultivating a probiotic *L. reuteri* strain in the presence of galacto-oligosaccharide (GOS) in a growth medium, thereby pre-conditioning the probiotic *L. reuteri* strain; and
ii) optionally harvesting the pre-conditioned probiotic *L. reuteri* strain from the growth medium; and
b) preparing a probiotic composition comprising the pre-conditioned probiotic *L. reuteri* strain obtained from step a)i) or a)ii) and adding GOS to the probiotic composition.

2. The method according to claim 1, wherein GOS is added in step a)i) to the growth medium in an amount of at least 0.2 wt%, preferably at least 0.5 wt%, even more preferably at least 0.75 wt% of GOS in the growth medium, and preferably at most 8 wt% of GOS, such as at most 7 wt% GOS, at most 6 wt% GOS, or even at most 5 wt% GOS, more preferably at most 4 wt% of GOS or even at most 3% GOS, such as in a range of 0.2 wt% to 8 wt% or even 0.2 wt% to 7 wt%, or 0.2 wt% to 6 wt%, more preferably in a range of 0.5 wt% to 8 wt% or even 0.5 wt% to 7 wt%, or 0.5 wt% to 6 wt%, even more preferably in a range of 0.75 wt% to 8 wt% or even 0.75 wt% to 7 wt%, or 0.75 wt% to 6 wt%, and most preferably in a range of 0.75 wt% to 7 wt%, in a range of 0.75 wt% to 6 wt% or even in a range of 0.75 wt% to 5 wt%, such as a range of 0.75 wt% to 3 wt% or 0.75 wt% to 4 wt%, preferably calculated on basis of the growth medium (% w/w) preferably at the start, in the middle or at the end of cultivation of the probiotic *L. reuteri* strain in step a)i).

3. The method according to claim 1 or 2, wherein GOS is added in step b) to the pre-conditioned probiotic *L. reuteri* strain obtained from step a)i) or a)ii) in an amount of at least 1 or 2 g per daily dose, preferably at least 3 g per daily dose, likewise preferably between 2 to 12 g per daily dose, or between 2 to 10 g per daily dose, between 2 to 8 g per daily dose, or between 2 to 7 g per daily dose, more preferably between 3 to 12 g per daily dose, likewise more preferably between 3 and 10 g per daily dose, such as between 3 to 8 g per daily dose, between 3 to 7 g per daily dose, between 3 to 6 g per daily dose, or about 2, 3, 4, 5, 6, 7, 8, 9, 10, 11 or 12 g per daily dose or any range formed thereby.

4. The method according to claim 1 to 3, wherein GOS added to the growth medium in step a)i) and/or to the composition comprising the pre-conditioned probiotic *L. reuteri* strain prepared in step b) has a degree of polymerization (DP) of 3-8.

5. The method according to claim any of claims 1 to 4, wherein the probiotic *L. reuteri* strain is at least one *L. reuteri* strain or multiple *L. reuteri* strains selected from the group comprising or consisting *of L. reuteri* strain as deposited under DSM 17938, *L. reuteri* strain as deposited under ATCC PTA 5289, *L. reuteri* strain as deposited under ATCC PTA 6475, *L. reuteri* as deposited under DSM 32846, *L. reuteri* as deposited under DSM 32848, *L. reuteri* as deposited under DSM 32849, *L. reuteri* as deposited under DSM 27131, , *L. reuteri* as deposited under DSM 32465 and *L. reuteri* strain as deposited under ATCC PTA 4659, preferably the probiotic *L. reuteri* strain is *L. reuteri* strain as deposited under DSM 17938.

6. A composition comprising:
an effective amount of a pre-conditioned probiotic *Lactobacillus reuteri* strain, wherein the pre-conditioned probiotic *L. reuteri* strain has been prepared by cultivating a *L. reuteri* strain in the presence of galacto-oligosaccharides (GOS) in a growth medium; and additionally
an effective amount of a GOS,
wherein the composition preferably has been prepared according to a method according to any of claims 1 to 5.

7. The composition according to claim 6, wherein the composition comprises the pre-conditioned probiotic *L. reuteri* strain in an amount of between 10³ cfu to 10¹² cfu, typically in an amount of between 10⁴ cfu to 10¹¹ cfu per daily dose, preferably in an amount of between 10⁵ cfu to 10¹⁰ cfu per daily dose, or 10⁵ cfu to 10⁹ cfu per daily dose, likewise preferably in an amount of between 10⁶ cfu to 10⁹ cfu per daily dose, 10⁶ cfu to 10⁸ cfu per daily dose or in an amount of 10⁸ cfu to 10¹⁰ cfu per daily dose, more preferably around 10⁷ cfu to 10⁹ cfu per daily dose, most preferably in an amount of around 10⁸ total cfu per daily dose, such as 10⁷ to 10⁹ cfu per daily dose or 10⁸ to 10⁹ cfu per daily dose.

8. The composition according to claim 6 or 7, wherein the composition comprises GOS in an amount of at least 1 or 2 g per daily dose, preferably at least 3 g per daily dose, likewise preferably between 2 to 12 g per daily dose, or between 2 to 10 g per daily dose, between 2 to 8 g per daily dose, or between 2 to 7 g per daily dose, more preferably between 3 to 12 g per daily dose, likewise more preferably between 3 and 10 g per daily dose, such as between 3 to 8 g per daily dose, between 3 to 7 g per daily dose, between 3 to 6 g per daily dose, or about 2, 3, 4, 5, 6, 7, 8, 9, 10, 11, or 12 g per daily dose or any range formed thereby.

9. The composition according to any of claims 6 to 8, wherein
the composition is solid or liquid, and/or is present in form of a powder, a tablet, a capsule, or may be in form of an oil formulation, an emulsion, an oil-in-water emulsion (o/w emulsion), or a water-in oil emulsion (w/o emulsion); and/or
the composition is an administrable form, preferably selected from the group consisting of nutritional compositions, pharmaceutical formulations, dietary supplements, functional food products, functional beverage products, and combinations thereof, e.g., a dairy product, a milk-based product, a whey protein-based beverage.

10. Non-therapeutic use of a composition comprising an effective amount of a pre-conditioned probiotic *Lactobacillus reuteri* strain supplemented with an effective amount of galacto-oligosaccharides (GOS), the composition being prepared according to any of claims 1 to 5 or is a composition according to any of claims 6 to 9, for improving or increasing the probiotic effect, preferably of a *L. reuteri* strain, in the digestive tract of a healthy subject.

11. The non-therapeutic use according to claim 10, wherein improving or increasing the probiotic effect:
- is an increase of mineral bio-accessibility, preferably an increase of mineral solubility and/or mineral absorption, in the intestinal tract of the healthy subject, wherein the mineral is preferably selected from calcium and/or iron; and/or
- is a promotion of a healthy microbiota in the intestinal tract of the healthy subject; and/or
- is a promotion of the survival of the *L. reuteri* strain in the colon and thus a promotion of the health benefits associated to the *L. reuteri* strain; and/or
- is an increase of metabolic activity of the gut microbiome such as an increase in the lactic acid/acetic acid production in the intestinal tract of the healthy subject; and/or
- is an improvement of bone health, bone strength, bone formation, teeth conditions, teeth growth, muscular functions, cognitive functions, immune functions and/or general growth and development (especially for children/infants) due to an increase of mineral solubility and/or mineral absorption, in the intestinal tract of the healthy subject.

12. A composition comprising an effective amount of a pre-conditioned probiotic *Lactobacillus reuteri* strain and an effective amount of galacto-oligosaccharides (GOS) as prepared according to any of claims 1 to 5 or according to any of claims 6 to 9 for use as a medicament.

13. A composition comprising an effective amount of a pre-conditioned probiotic *Lactobacillus reuteri* strain and an effective amount of galacto-oligosaccharides (GOS) as prepared according to any of claims 1 to 5 or according to any of claims 6 to 9 for use in the prevention and/or treatment of mineral deficiency in a patient in need thereof, wherein the patient is preferably at risk of developing mineral deficiency or is a subject suffering from mineral deficiency, wherein the mineral is optionally selected from calcium and/or iron.

14. The composition for use according to claim 13, wherein the composition is for use in the prevention and/or treatment of:
iron deficiency, preferably iron deficiency anemia, and/or promote cognitive functions, and/or promote oxygen transport, and/or promote immune functions, and/or reduces tiredness or fatigue, in a patient in need thereof; and/or calcium deficiency in a patient in need thereof.

15. The composition for use according to claim 13 or 14, wherein the composition is for use in the prevention and/or treatment of a digestive dysfunction, an impaired microbiota, and/or a digestive dysfunction.

16. A composition comprising an effective amount of a pre-conditioned probiotic *Lactobacillus reuteri* strain and an effective amount of galacto-oligosaccharides (GOS) as prepared according to any of claims 1 to 5 or according to any of claims 6 to 9 for use in the prevention and/or treatment of an impaired microbiota or a dysbiosis.

17. A composition comprising an effective amount of a pre-conditioned probiotic *Lactobacillus reuteri* strain and an effective amount of galacto-oligosaccharides (GOS) as prepared according to any of claims 1 to 5 or according to any of claims 6 to 9 for use in the prevention and/or treatment of bone loss and/or to promote growth and development of bones or teeth in children, and/or promote muscular functions, and/or promote functions of the nervous system and/or bone or muscular related conditions in a patient in need thereof.

18. A composition comprising an effective amount of a pre-conditioned probiotic *Lactobacillus reuteri* strain and an effective amount of galacto-oligosaccharides (GOS) as prepared according to any of claims 1 to 5 or according to any of claims 6 to 9 for use in the prevention and/or treatment of infections, preferably gut infections, and/or to promote immune functions, optionally through the increase of the lactic acid/acetic acid production in the microbiota of the patient.
